# EUROPEAN PATENT APPLICATION

(11) **EP 4 458 851 A1**
(43) Date of publication of application: **06.11.2024**
(21) Application number: 22914978.6
(22) Date of filing: 28.12.2022
(51) Int. Cl.: C07K 16/28, A61K 39/395, A61P 35/02

(54) **ANTI-GPRC5D ANTIBODY AND USE THEREOF**

(30) Priority: 31.12.2021 CN 202111663745
(71) Applicant: Kyinno Biotechnology Co., Ltd., Beijing 101111 (CN)
(72) Inventor: NING, Jinying, Beijing 101111 (CN); PENG, Hao, Beijing 101111 (CN); HAO, Feng, Beijing 101111 (CN); HE, Feng, Beijing 101111 (CN)
(74) Representative: Cabinet Laurent & Charras
(86) International application number: PCT/CN2022/143042
(87) International publication number: WO 2023/125728

(57) **Abstract**

Provided is an anti-G protein-coupled receptor family class C group 5 member D (GPRC5D) antibody or a fragment thereof. Also provided is the use of the antibody or fragment thereof as an active ingredient for treatment of tumors or cancers. In addition, further provided is a bispecific antibody comprising the antibody or fragment thereof and aiming at GPRC5D and CD3.

## Description

### Cross Reference of related Application

This application claims the priority of Chinese Application No. CN202111663745.2, filed December 31, 2021, which is hereby incorporated by reference in their entirety.

### Technical Field

The present disclosure belongs to the field of biomedicine and relates to novel anti-GPRC5D antibodies or functional fragments thereof. The present disclosure also relates to use of the antibodies or functional fragments thereof.

### Background

G protein-coupled receptor class C group 5 member D (GPRC5D) is a retinoic acid induced 40kDa protein having 7 transmembrane segments and a short N-terminal extracellular region. It is expressed in keratinized epithelial cells, pancrea, kidney, small intestine, spleen and testis and the like. In March 2019, the journal of Science Translational Medicine published a research article on the treatment of multiple myeloma with CAR-T targeting GPRC5D, preliminarily showing the potential of GPRC5D as a new target for the treatment of multiple myeloma. Tissue expression profiling studies have found that GPRC5D is specifically highly expressed in plasma cells in multiple myeloma. In addition, it has been proposed that the disease associated with GPRC5D includes chromosome 13Q14 deletion syndrome and the like.

Given the significant role of GPRC5D in diseases including multiple myeloma, there is a need to identify more anti-GPRC5D antibodies.

### Summary

In view of the above technical problems, herein provided are antibodes or fragments thereof against G protein-coupled receptor class C group 5 member D (GPRC5D), and the use based on the antibodies or fragments thereof. In addition, herein provided is bispecific antibodies directed to GPRC5D and CD3 based on the antibodies or fragments thereof.

The present disclosure provides the following technical solutions.

In one aspect, the present disclosure provides an antibody or a fragment thereof binding specifically to GPRC5D, in particular human GPRC5D.

In an embodiment, the antibody or fragment thereof provided by the present disclosure comprises a variable region of a heavy chain (VH) and a variable region of a light chain (VL), wherein the variable region of the heavy chain (VH) and the variable region of the light chain (VL) comprise a CDR combination of H-CDR1, H-CDR2, H-CDR3, L-CDR1, L-CDR2 and L-CDR3 selected from the following:
(1) H-CDR1 comprising an amino acid sequence set forth in SEQ ID NO: 28 (SDYAWN), H-CDR2 comprising an amino acid sequence set forth in SEQ ID NO: 29 (YISYSGSATYNPSLKS), H-CDR3 comprising an amino acid sequence set forth in SEQ ID NO: 30 (GGIAGRGRWGAMDY); and L-CDR1 comprising an amino acid sequence set forth in SEQ ID NO: 31 (KASQNVGTNVA), L-CDR2 comprising an amino acid sequence set forth in SEQ ID NO: 32 (SASYRDS), L-CDR3 comprising an amino acid sequence set forth in SEQ ID NO: 33 (QQYKSYPLT);
(2) H-CDR1 comprising an amino acid sequence set forth in SEQ ID NO: 28 (SDYAWN), H-CDR2 comprising an amino acid sequence set forth in SEQ ID NO: 34 (YISYSGSATYSPSLKS), H-CDR3 comprising an amino acid sequence set forth in SEQ ID NO: 30 (GGIAGRGRWGAMDY); and L-CDR1 comprising an amino acid sequence set forth in SEQ ID NO: 31 (KASQNVGTNVA), L-CDR2 comprising an amino acid sequence set forth in SEQ ID NO: 32 (SASYRDS), L-CDR3 comprising an amino acid sequence set forth in SEQ ID NO: 33 (QQYKSYPLT);
(3) H-CDR1 comprising an amino acid sequence set forth in SEQ ID NO: 35 (SYTIQ), H-CDR2 comprising an amino acid sequence set forth in SEQ ID NO: 36 (YIIPSSGYTNYNQKFKD), H-CDR3 comprising an amino acid sequence set forth in SEQ ID NO: 37 (NYGNWGFTY); and L-CDR1 comprising an amino acid sequence set forth in SEQ ID NO: 38 (KASQNVGSAVT), L-CDR2 comprising an amino acid sequence set forth in SEQ ID NO: 39 (SASNRYT), L-CDR3 comprising an amino acid sequence set forth in SEQ ID NO: 40 (QQYSNYPLT);
(4) H-CDR1 comprising an amino acid sequence set forth in SEQ ID NO: 41 (YYVMH), H-CDR2 comprising an amino acid sequence set forth in SEQ ID NO: 42 (YINPYNDGTKYNEKFKG), H-CDR3 comprising an amino acid sequence set forth in SEQ ID NO: 43 (GGVRRYFDY); and L-CDR1 comprising an amino acid sequence set forth in SEQ ID NO: 44 (RASQDIGSNLN), L-CDR2 comprising an amino acid sequence set forth in SEQ ID NO: 45 (ATSSLDS), L-CDR3 comprising an amino acid sequence set forth in SEQ ID NO: 46 (LQYATFPYT);
(5) H-CDR1 comprising an amino acid sequence set forth in SEQ ID NO: 47 (YYVIH), H-CDR2 comprising an amino acid sequence set forth in SEQ ID NO: 42 (YINPYNDGTKYNEKFKG), H-CDR3 comprising an amino acid sequence set forth in SEQ ID NO: 43 (GGVRRYFDY); and L-CDR1 comprising an amino acid sequence set forth in SEQ ID NO: 44 (RASQDIGSNLN), L-CDR2 comprising an amino acid sequence set forth in SEQ ID NO: 45 (ATSSLDS), L-CDR3 comprising an amino acid sequence set forth in SEQ ID NO: 46 (LQYATFPYT); and
(6) H-CDR1 comprising an amino acid sequence set forth in SEQ ID NO: 47 (YYVIH), H-CDR2 comprising an amino acid sequence set forth in SEQ ID NO: 48 (YINPYNAGTKYNEKFKG), H-CDR3 comprising an amino acid sequence set forth in SEQ ID NO: 43 (GGVRRYFDY); and L-CDR1 comprising an amino acid sequence set forth in SEQ ID NO: 44 (RASQDIGSNLN), L-CDR2 comprising an amino acid sequence set forth in SEQ ID NO: 45 (ATSSLDS), L-CDR3 comprising an amino acid sequence set forth in SEQ ID NO: 46 (LQYATFPYT).

The CDR combination of the light and heavy chains provided in the context of the present disclosure are all from the antibody or fragment thereof of the present disclosure, and a person skilled in the art would routinely determine the CDRs contained therein based on the variable region amino acid sequence comprised in a given antibody or fragment thereof. For example, according to a specific embodiment of the present disclosure, the KABAT method is used to define the CDRs in the variable region amino acid sequence.

In the antibody or fragment thereof provided in the present disclosure, preferably, the variable region of the heavy chain comprises a sequence selected from an amino acid sequence set forth in or an amino acid sequence having at least 75% identity with SEQ ID NO: 5, SEQ ID NO: 13, SEQ ID NO: 7, SEQ ID NO: 15, SEQ ID NO: 9, SEQ ID NO: 17, SEQ ID NO: 11 or SEQ ID NO: 19; and/or, the variable region of the light chain comprises a sequence selected from an amino acid sequence set forth in or an amino acid sequence having at least 75% identity with SEQ ID NO: 6, SEQ ID NO: 14, SEQ ID NO: 8, SEQ ID NO: 16, SEQ ID NO: 10, SEQ ID NO: 18, SEQ ID NO: 12 or SEQ ID NO: 20.

More preferably, the variable region of the heavy chain and the variable region of the light chain in the antibody or fragment thereof comprise a sequence combination selected from the following:
(1) an amino acid sequence set forth in or an amino acid sequence having at least 75% identity with SEQ ID NO: 5; and an amino acid sequence set forth in or an amino acid sequence having at least 75% identity with SEQ ID NO: 6;
(2) an amino acid sequence set forth in or an amino acid sequence having at least 75% identity with SEQ ID NO: 13; and an amino acid sequence set forth in or an amino acid sequence having at least 75% identity with SEQ ID NO: 14;
(3) an amino acid sequence set forth in or an amino acid sequence having at least 75% identity with SEQ ID NO: 7; and an amino acid sequence set forth in or an amino acid sequence having at least 75% identity with SEQ ID NO: 8;
(4) an amino acid sequence set forth in or an amino acid sequence having at least 75% identity with SEQ ID NO: 15; and an amino acid sequence set forth in or an amino acid sequence having at least 75% identity with SEQ ID NO: 16;
(5) an amino acid sequence set forth in or an amino acid sequence having at least 75% identity with SEQ ID NO: 9; and an amino acid sequence set forth in or an amino acid sequence having at least 75% identity with SEQ ID NO: 10;
(6) an amino acid sequence set forth in or an amino acid sequence having at least 75% identity with SEQ ID NO: 17; and an amino acid sequence set forth in or an amino acid sequence having at least 75% identity with SEQ ID NO: 18;
(7) an amino acid sequence set forth in or an amino acid sequence having at least 75% identity with SEQ ID NO: 11; and an amino acid sequence set forth in or an amino acid sequence having at least 75% identity with SEQ ID NO: 12; and
(8) an amino acid sequence set forth in or an amino acid sequence having at least 75% identity with SEQ ID NO: 19; and an amino acid sequence set forth in or an amino acid sequence having at least 75% identity with SEQ ID NO: 20.

In the context of the present disclosure, "at least 75% identity" is identity of any percentage between 75% and 100%, for example 75%, 80%, 85%, 90%, or even 91%, 92%, 93%, 94%, 95%, 96%, 97%, 98%, 99% or even 100% identity.

In an embodiment, the antibody or fragment thereof of the present disclosure comprises at least a variable region of a heavy chain and a variable region of a light chain, both of which comprise the above-mentioned CDRs and a framework region in between, and each domain is arranged as: FR1-CDR1-FR2-CDR2-FR3-CDR3-FR4. Moreover, optionally, the at most 25% difference in an amino acid sequence resulted from the "at least 75% identity" may exist in any framework region in the variable region of the heavy chain or the variable region of the light chain, or in any domain or any sequence other than the variable region of the heavy chain and the variable region of the light chain in the antibody or fragment thereof of the present disclosure. The difference may be caused by deletion, addition or substitution of an amino acid at any position.

In terms of antigen, the antibody or fragment thereof of the present disclosure is an antibody or fragment thereof against GPRC5D; preferably, the antibody is in any form, such as a murine antibody, a rabbit antibody, a monoclonal antibody, a chimeric antibody, a partially or fully humanized antibody, or the fragment is a half antibody or an antigen-binding fragment of an antibody or a half antibody, such as scFv, BsFv, dsFv, (dsFv)₂, Fab, Fab', F(ab')₂ or Fv; more preferably, the antibody is IgG. In an embodiment, herein provides an isolated and structurally-characterized human antibody, such as a human monoclonal antibody, that specifically binds to GPRC5D (such as human GPRC5D).

In addition to the variable region, an antibody or fragment thereof provided by the present disclosure further comprises a constant region, such as a human or murine constant region, preferably, the antibody or fragment thereof comprises a human or murine heavy chain constant region (CH) and/or light chain constant region (CL); preferably, the antibody or fragment thereof comprises a heavy chain and a light chain; more preferably, the antibody or fragment thereof comprises a heavy chain constant region of IgG, IgA, IgM, IgD or IgE and/or a κ or λ light chain constant region.

In an embodiment, the antibody is a monoclonal antibody, preferably a murine, a chimeric or a humanized monoclonal antibody. Preferably, the heavy chain constant region of the monoclonal antibody is of IgG1 or IgG4 subtype, and the light chain constant region is of κ type. Preferably, the heavy chain constant region of the monoclonal antibody comprises an amino acid sequence set forth in or an amino acid sequence having at least 75% identity with SEQ ID NO: 23; the light chain constant region of the monoclonal antibody comprises an amino acid sequence set forth in or an amino acid sequence having at least 75% identity with SEQ ID NO: 24.

In an embodiment, the anti-human GPRC5D antibody is a monoclonal antibody. Preferably, the anti-human GPRC5D antibody is an immunoglobulin, for example, the immunoglobulin is of human IgA, IgD, IgE, IgG or IgM type. Moreover, preferably, the antibody is of human IgG1 or IgG4 subtype.

The antibody or fragment thereof provided herein may also be used to construct an anti-GPRC5D×CD3 bispecific antibody. Therefore, the present disclosure also provides the following bispecific antibody construct.

The present disclosure provies a bispecific antibody construct comprising a first binding domain that binds to G protein-coupled receptor class C group 5 member D (GPRC5D), and a second binding domain that binds to CD3 on the surface of a T cell.

In a bispecific antibody construct provided herein, the first binding domain comprises a variable region of a heavy chain (VH) and a variable region of a light chain (VL), wherein the variable region of the heavy chain (VH) and the variable region of the light chain (VL) comprise a CDR combination of H-CDR1, H-CDR2, H-CDR3, L-CDR1, L-CDR2, and L-CDR3 selected from the following:
(1) H-CDR1 comprising an amino acid sequence set forth in SEQ ID NO: 28 (SDYAWN), H-CDR2 comprising an amino acid sequence set forth in SEQ ID NO: 29 (YISYSGSATYNPSLKS), H-CDR3 comprising an amino acid sequence set forth in SEQ ID NO: 30 (GGIAGRGRWGAMDY); and L-CDR1 comprising an amino acid sequence set forth in SEQ ID NO: 31 (KASQNVGTNVA), L-CDR2 comprising an amino acid sequence set forth in SEQ ID NO: 32 (SASYRDS), L-CDR3 comprising an amino acid sequence set forth in SEQ ID NO: 33 (QQYKSYPLT);
(2) H-CDR1 comprising an amino acid sequence set forth in SEQ ID NO: 28 (SDYAWN), H-CDR2 comprising an amino acid sequence set forth in SEQ ID NO: 34 (YISYSGSATYSPSLKS), H-CDR3 comprising an amino acid sequence set forth in SEQ ID NO: 30 (GGIAGRGRWGAMDY); and L-CDR1 comprising an amino acid sequence set forth in SEQ ID NO: 31 (KASQNVGTNVA), L-CDR2 comprising an amino acid sequence set forth in SEQ ID NO: 32 (SASYRDS), L-CDR3 comprising an amino acid sequence set forth in SEQ ID NO: 33 (QQYKSYPLT);
(3) H-CDR1 comprising an amino acid sequence set forth in SEQ ID NO: 35 (SYTIQ), H-CDR2 comprising an amino acid sequence set forth in SEQ ID NO: 36 (YIIPSSGYTNYNQKFKD), H-CDR3 comprising an amino acid sequence set forth in SEQ ID NO: 37 (NYGNWGFTY); and L-CDR1 comprising an amino acid sequence set forth in SEQ ID NO: 38 (KASQNVGSAVT), L-CDR2 comprising an amino acid sequence set forth in SEQ ID NO: 39 (SASNRYT), L-CDR3 comprising an amino acid sequence set forth in SEQ ID NO: 40 (QQYSNYPLT);
(4) H-CDR1 comprising an amino acid sequence set forth in SEQ ID NO: 41 (YYVMH), H-CDR2 comprising an amino acid sequence set forth in SEQ ID NO: 42 (YINPYNDGTKYNEKFKG), H-CDR3 comprising an amino acid sequence set forth in SEQ ID NO: 43 (GGVRRYFDY); and L-CDR1 comprising an amino acid sequence set forth in SEQ ID NO: 44 (RASQDIGSNLN), L-CDR2 comprising an amino acid sequence set forth in SEQ ID NO: 45 (ATSSLDS), L-CDR3 comprising an amino acid sequence set forth in SEQ ID NO: 46 (LQYATFPYT);
(5) H-CDR1 comprising an amino acid sequence set forth in SEQ ID NO: 47 (YYVIH), H-CDR2 comprising an amino acid sequence set forth in SEQ ID NO: 42 (YINPYNDGTKYNEKFKG), H-CDR3 comprising an amino acid sequence set forth in SEQ ID NO: 43 (GGVRRYFDY); and L-CDR1 comprising an amino acid sequence set forth in SEQ ID NO: 44 (RASQDIGSNLN), L-CDR2 comprising an amino acid sequence set forth in SEQ ID NO: 45 (ATSSLDS), L-CDR3 comprising an amino acid sequence set forth in SEQ ID NO: 46 (LQYATFPYT); and
(6) H-CDR1 comprising an amino acid sequence set forth in SEQ ID NO: 47 (YYVIH), H-CDR2 comprising an amino acid sequence set forth in SEQ ID NO: 48 (YINPYNAGTKYNEKFKG), H-CDR3 comprising an amino acid sequence set forth in SEQ ID NO: 43 (GGVRRYFDY); and L-CDR1 comprising an amino acid sequence set forth in SEQ ID NO: 44 (RASQDIGSNLN), L-CDR2 comprising an amino acid sequence set forth in SEQ ID NO: 45 (ATSSLDS), L-CDR3 comprising an amino acid sequence set forth in SEQ ID NO: 46 (LQYATFPYT).

Preferably, in a first binding domain, the variable region of the heavy chain comprises a sequence selected from an amino acid sequence set forth in or an amino acid sequence having at least 75% identity with SEQ ID NO: 5, SEQ ID NO: 13, SEQ ID NO: 7, SEQ ID NO: 15, SEQ ID NO: 9, SEQ ID NO: 17, SEQ ID NO: 11 or SEQ ID NO: 19; and/or, the variable region of the light chain comprises a sequence selected an amino acid sequence set forth in or an amino acid sequence having at least 75% identity with SEQ ID NO: 6, SEQ ID NO: 14, SEQ ID NO: 8, SEQ ID NO: 16, SEQ ID NO: 10, SEQ ID NO: 18, SEQ ID NO: 12 or SEQ ID NO: 20.

More preferably, the variable region of the heavy chain and the variable region of the light chain in the first binding domain comprise a sequence combination selected from the following:
(1) an amino acid sequence set forth in or an amino acid sequence having at least 75% identity with SEQ ID NO: 5; and an amino acid sequence set forth in or an amino acid sequence having at least 75% identity with SEQ ID NO: 6;
(2) an amino acid sequence set forth in or an amino acid sequence having at least 75% identity with SEQ ID NO: 13; and an amino acid sequence set forth in or an amino acid sequence having at least 75% identity with SEQ ID NO: 14;
(3) an amino acid sequence set forth in or an amino acid sequence having at least 75% identity with SEQ ID NO: 7; and an amino acid sequence set forth in or an amino acid sequence having at least 75% identity with SEQ ID NO: 8;
(4) an amino acid sequence set forth in or an amino acid sequence having at least 75% identity with SEQ ID NO: 15; and an amino acid sequence set forth in or an amino acid sequence having at least 75% identity with SEQ ID NO: 16;
(5) an amino acid sequence set forth in or an amino acid sequence having at least 75% identity with SEQ ID NO: 9; and an amino acid sequence set forth in or an amino acid sequence having at least 75% identity with SEQ ID NO: 10;
(6) an amino acid sequence set forth in or an amino acid sequence having at least 75% identity with SEQ ID NO: 17; and an amino acid sequence set forth in or an amino acid sequence having at least 75% identity with SEQ ID NO: 18;
(7) an amino acid sequence set forth in or an amino acid sequence having at least 75% identity with SEQ ID NO: 11; and an amino acid sequence set forth in or an amino acid sequence having at least 75% identity with SEQ ID NO: 12; or
(8) an amino acid sequence set forth in or an amino acid sequence having at least 75% identity with SEQ ID NO: 19; and an amino acid sequence set forth in or an amino acid sequence having at least 75% identity with SEQ ID NO: 20.

In a bispecific antibody construct provided herein, the second binding domain comprises a variable region of a heavy chain (VH) and a variable region of a light chain (VL), wherein the variable region of the heavy chain (VH) and the variable region of the light chain (VL) comprise a CDR combination of H-CDR1, H-CDR2, H-CDR3, L-CDR1, L-CDR2, and L-CDR3 selected from the following:
H-CDR1 comprising an amino acid sequence set forth in SEQ ID NO: 49 (TYAMN), H-CDR2 comprising an amino acid sequence set forth in SEQ ID NO: 50 (RIRSKYNNYATYYADSVKD), H-CDR3 comprising an amino acid sequence set forth in SEQ ID NO: 51 (HGNFGNSYVSYFAY); and L-CDR1 comprising an amino acid sequence set forth in SEQ ID NO: 52 (RSSTGAVTTSNYAN), L-CDR2 comprising an amino acid sequence set forth in SEQ ID NO: 53 (GTNKRAP), L-CDR3 comprising an amino acid sequence set forth in SEQ ID NO: 54 (ALWYSNLWV).

Preferably, in a second binding domain, the variable region of the heavy chain comprises an amino acid sequence set forth in or an amino acid sequence having at least 75% identity with SEQ ID NO: 21; and the variable region of the light chain comprises an amino acid sequence set forth in or an amino acid sequence having at least 75% identity with SEQ ID NO: 22.

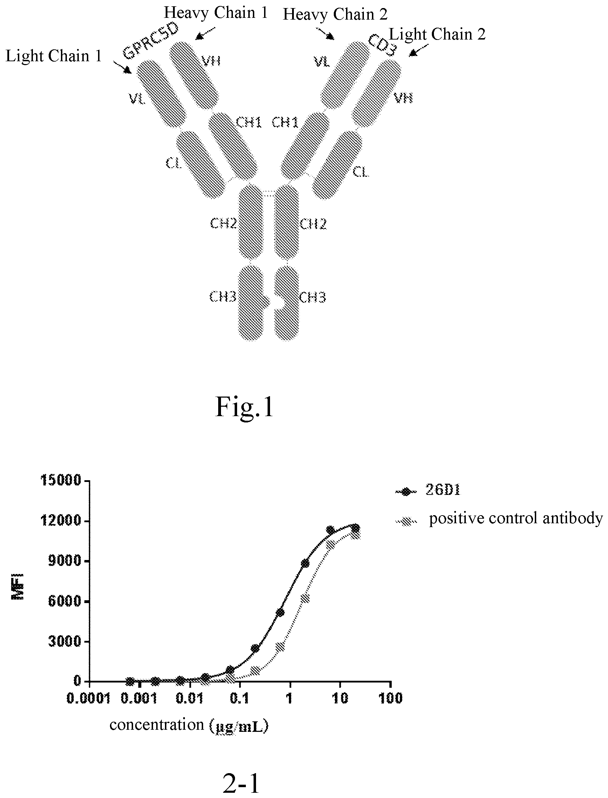

Further, preferably, a bispecific antibody construct provided herein comprises four polypeptide chains:
1) Heavy Chain 1 comprising domains arranged as VH-CH1-CH2-CH3 from N-terminus to C-terminus;
2) Heavy Chain 2 comprising domains arranged as VL-CH1-CH2-CH3 from N-terminus to C-terminus;
3) Light Chain 1 comprising domains arranged as VL-CL from N-terminus to C-terminus;
4) Light Chain 2 comprising domains arranged as VH-CL from N-terminus to C-terminus;
wherein the VH in Heavy Chain 1 and the VL in Light Chain 1 are paired to form the first binding domain that binds to GPRC5D; the VL in Heavy Chain 2 and the VH in Light Chain 2 are paired to form the second binding domain that binds to CD3. The structural schematic diagram of a bispecific antibody construct provided herein is illustrated in Figure 1.

In an embodiment, in the bispecific antibody construct provided herein, the CH1-CH2-CH3 domain in Heavy Chain 1 comprises an amino acid sequence set forth in or an amino acid sequence having at least 75% identity with SEQ ID NO: 25; the CH1-CH2-CH3 domain in Heavy Chain 2 comprises an amino acid sequence set forth in or an amino acid sequence having at least 75% identity with SEQ ID NO: 26; the CL domain in Light Chain 1 comprises an amino acid sequence set forth in or an amino acid sequence having at least 75% identity with SEQ ID NO: 24; and the CL domain in Light Chain 2 comprises an amino acid sequence set forth in or an amino acid sequence having at least 75% identity with SEQ ID NO: 27.

In another aspect, the present disclosure provides a nucleic acid molecule comprising a nucleotide sequence encoding a heavy chain CDR, a light chain CDR, a light chain variable region, a heavy chain variable region, a heavy chain or a light chain comprised in an antibody or fragment thereof or a bispecific antibody construct provided in the present disclosure.

The nucleic acid molecule of the present disclosure may be cloned into a vector, and then transformed or transfected into a host cell. Therefore, in another aspect, the present disclosure provides a vector comprising a nucleic acid molecule of the present disclosure. The vector can be a eukaryotic expression vector, a prokaryotic expression vector, an artificial chromosome and a phage vector, etc. The vector or nucleic acid molecule herein can be used to transform or transfect a host cell. Therefore, in another aspect, the present disclosure provides a host cell comprising a nucleic acid molecule and/or a vector of the present disclosure, or transformed or transfected by the nucleic acid molecule and/or the vector of the present disclosure. The host cell can be any prokaryotic or eukaryotic cell, such as a bacterium or an insect, fungus, plant or animal cell.

The antibody or fragment thereof and the bispecific antibody construct provided in the present disclosure may be obtained by any method known in the art. For example, the host cell is cultivated in a condition where the host cell is allowed to express the heavy chain and light chain of the antibody. Optionally, the method also comprises the step of recycling the antibody produced.

The antibody or fragment thereof, the bispecific antibody construct, the nucleic acid molecule, the vector and/or the host cell of the present disclosure may be comprised in a composition, more particularly in a pharmaceutical composition such as a pharmaceutical formulation, so as to be used for various purposes according to actual needs. Therefore, in another aspect, the present disclosure provides a composition, such as a pharmaceutical composition, comprising an antibody or fragment thereof, a bispecific antibody construct, a nucleic acid molecule, a vector and/or a host cell described in the present disclosure, and optionally a pharmaceutically acceptable excipient.

In another aspect, the present disclosure provides use of an antibody or fragment thereof, a bispecific antibody construct, a nucleic acid molecule, a vector, a host cell and/or a composition of the present disclosure in the preparation of a medicament for treating a disease, wherein the disease is a tumor or cancer. Preferably, the disease is a tumor or cancer associated with GPRC5D expression (e.g., high expression), such as lymphoma or myeloma, particullarly multiple myeloma.

Accordingly, the present disclosure provides a method for treating a disease comprising administering an antibody or fragment thereof, a bispecific antibody construct, a nucleic acid molecule, a vector, a host cell and/or a composition of the present disclosure to a subject in need thereof, wherein the disease is a tumor or cancer. Preferably, the disease is a tumor or cancer associated with GPRC5D expression (e.g., high expression), such as lymphoma or myeloma; particullarly multiple myeloma. Preferably, the subject is a mammal; more preferably, the subject is human.

In another aspect, the present disclosure provides use of an antibody or fragment thereof, a bispecific antibody construct, a nucleic acid molecule, a vector, a host cell and/or a composition of the present disclosure in the preparation of a reagent for diagnosing a disease or detecting the presence of antigen GPRC5D, wherein the disease is a tumor or cancer. Preferably, the disease is a tumor or cancer associated with GPRC5D expression (e.g., high expression), such as lymphoma or myeloma, particullarly multiple myeloma.

In another aspect, the present disclosure provides a kit comprising an antibody or fragment thereof, a bispecific antibody construct, a nucleic acid molecule, a vector, a host cell and/or a composition of the present disclosure. The kit can be used for the treatment mentioned above, or for diagnosis or antigen detection. Depending on applications of interest, the kit also comprises an additional reagent for treatment, diagnosis or detection. For example, the kit is one for detecting the presence of GPRC5D in any biological sample using ELISA.

### BRIEF DESCRIPTION OF THE DRAWINGS

The embodiments of the present disclosure are described in detail below with reference to the accompanying drawings, wherein:
Figure 1 is a schematic diagram of the structure of an anti-hGPRC5D×CD3 bispecific antibody.
Figure 2 shows the FACS detection results of the hybridoma cell supernatant binding to different cells.
Figure 3 shows the FACS detection results of the hybridoma cell supernatant binding to different cells.
Figure 4 shows the FACS detection results of the hybridoma cell supernatant binding to different cells, wherein 4-1: MM1R cells; 4-2: Nalm6 cells; 4-3: OPM2 cells; 4-4: NCI-H929 cells.
Figure 5 shows the FACS detection results of the humanized antibody binding to human GPRC5D expressing cells, wherein 5-1: 9D7(hz); 5-2: 26D1(hz); 5-3: 24F5(hz); 5-4: 6E97(hz).
Figure 6 shows the FACS detection results of the humanized antibody binding to monkey GPRC5D expressing cells, wherein 6-1: 9D7(hz); 6-2: 26D1(hz); 6-3: 24F5(hz); 6-4: 6E97(hz).
Figure 7 shows the FACS detection results of the humanized antibody binding to cells of different tumor cell lines, wherein 7-1: MM.1R cells; 7-2: MOLP8 cells.
Figure 8 shows the affinity test results of the humanized antibody to GPRC5D, wherein 8-1: GPRC5D×CD3 JNJ; 8-2: 9D7(hz); 8-3: 26D1(hz); 8-4: 24F5(hz); 8-5: 6E97(hz).
Figure 9 shows the detection results of humanized antibody-mediated ADCC, wherein 9-1: wild-type NCI-H929; 9-2: GPRC5D-knockout NCI-H929.
Figure 10 shows the detection results of bispecific antibody-mediated T cell killing, wherein 10-1: co-culture experiment 1; 10-2: co-culture experiment 2; 10-3: co-culture experiment 3.

### Detailed Description

The present disclosure is illustrated below with reference to examples. It will be appreciated by those skilled in the art that these examples are only used to illustrate the present disclosure and are not intended to limit the scope of the present disclosure in any way.

The experimental methods in the following examples are conventional methods unless otherwise specified. The materials, reagents, etc. used in the examples are commercially available products unless otherwise specified.

Human GPRC5D protein: Genbank accession number NM_018654;
Monkey GPRC5D protein: Genbank accession number XM _005570193.2.

### Example 1: Preparation of hybridoma cells and collection of antibody proteins

Cells expressing human GPRC5D (KYINNO BIOTECHNOLOGY CO., LTD., catalog number KC-1583) were prepared into a cell suspension at concentration of 1×10⁷ cells/ml in PBS with 2% FBS.

The cell suspension was used as immunogen to immunize mice. Five mice were immunized subcutaneously and five mice were immunized intramuscularly. The adjuvant used was quick antibody 5W water-soluble adjuvant. The titer was measured 2 weeks after the booster immunization. Two mice with high titers were selected for immune challenge. Serum and the spleen were collected after 3 days. The isolated spleen cells were fused with myeloma cells in culture in a 96-well plate, and selective culture medium was added for screening. The medium was changed after 7 days, and a cell-based ELISA test was performed after 10 days. 293T cells expressing human GPRC5D (Kyinno, catalog number KC-1723) were used for ELISA detection. Cells with OD value more than 10 times greater than that of the negative control (normal mouse serum) were selected for flow cytometry. 293T cells expressing human GPRC5D (Kyinno, catalog number KC-1723) were used for flow cytometry.

Cells positive in both ELISA and FACS assays were selected and subclone plated by the limited dilution method. Monoclonal cells were selected and the culture supernatant was taken for ELISA and flow cytometry. Double-positive cells were selected for expansion in culture.

The culture supernatant of the obtained hybridoma cells was collected, centrifuged, concentrated, and purified using G protein affinity column. Then, the purified sample was diluted 10 times with 20mM citric acid plus 100mM NaCl buffer pH 5.5, concentrated into a suitable volume via ultrafiltration, then dispensed into 1.5mL EP tubes, 200 µL/tube, and stored at -80°C. At the same time, some samples were taken for SDS-PAG and FACS assays to verify the purity and activity of the proteins.

### Example 2: ELISA detection of the binding between hybridoma cell culture supernatant and human GPRC5D

BXPC-3 cells expressing human GPRC5D (Kyinno, catalog number KC-1717) were prepared into a suspension at concentration of 1 × 10⁵ cells/ml in PBS containing 2% FBS. The suspension was added to a 96-well plate at 100 µl/well and cultured overnight at 37°C. The wells were cleared of their contents and washed three times with washing solution. 80µl of 4% tissue fixative (Beyotime, catalog number P0099) was added to each well followed by fixing at room temperature for 15min, washing twice with PBS and drying. 250µl of blocking solution containing 2% BSA was added to each well followed by incubation at 37°C for 1 hr and washing three times with PBS. 50-100µl of the supernatant of the hybridoma cell culture was added to each well. A positive control (adding serum from fusion mice), a negative control (adding serum from normal mice) and a blank control (adding culture medium) were included. The wells were incubated at 37°C for 1-2 hrs, washed, and dried. Then, the enzyme-labeled secondary antibody, HRP-labeled goat anti-mouse IgG (SIGMA, catalog number A9044-2ml) was added at dilution of 1:10000 and 50-100 µl/well. The wells were incubated at 37°C for 1-2 hrs, washed, and dried. 50-100µl of freshly prepared developing solution TMB was added to each well and incubated at 37°C for 10-30 mins.

2 mol/L H₂SO₄ was added to terminate the reaction, and the OD value was read on an enzyme-linked immunosorbent reader.

Results: P/N>2: 1 (P represents the positive value, N represents the value from normal mouse serum) is considered positive. If the negative control well is colorless or nearly colorless, and the positive control well is clearly colored, then the result can be observed visually. The results were shown in Table 1.

**Table 1 ELISA results of hybridoma cell culture supernatant binding to human GPRC5D**

| Sample shown by hybridoma cell number | OD value |
|---|---|
| 9D7 | 0.541 |
| 26D1 | 0.871 |
| 24F5 | 1.541 |
| 6E97 | 1.55 |
| 2G5 | 0.873 |
| 57G4 | 0.586 |
| Positive Control | 0.619 |
| Negative control | 0.238 |
| Blank control | 0.157 |

### Example 3: Construction of an anti-hGPRC5D×CD3 bispecific antibody

An anti-hGPRC5D×CD3 bispecific antibody was constructed using the GPRC5D-targeting domain and the CD3-targeting domain of Talquetamab (Johnson & Johnson, JNJ-64407564). The schematic diagram of the structure of the bispecific antibody was shown in Figure 1, where the domains comprised in each chain from the N-terminus to the C-terminus are as follows:
Heavy Chain 1: VH-CH1-CH2-CH3
   > VH (SEQ ID NO: 1) 
QVQLVQSGAEVKKPGASVKVSCKASGYSFTGYTMNWVRQAPGQGLEWMGLINPYNS
DTNYAQKLQGRVTMTTDTSTSTAYMELRSLRSDDTAVYYCARVALRVALDYWGQGTL
VTVSS
   > CH1-CH2-CH3 (SEQ ID NO: 25) 
ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPPAA
GPSVFLFPPKPKDTLMISRTPEVTCVVVDVSHEDPEVKFNWYVDGVEVHNAKTKPREE
QYNSTYRVVSVLTVLHQDWLNGKEYKCKVSNKALAAPIEKTISKAKGQPREPQVYTL
PPCREEMTKNQVSLWCLVKGFYPSDIAVEWESNGQPENNYKTTPPVLDSDGSFLLYSK
LTVDKSRWQQGNVFSCSVMHEALHNHYTQKSLSLSPGK
Heavy Chain 2: VL-CH1-CH2-CH3
   > VL (SEQ ID NO: 2) 
QTVVTQEPSLTVSPGGTVTLTCRSSTGAVTTSNYANWVQQKPGQAPRGLIGGTNKRAP
GTPARFSGSLLGGKAALTLSGVQPEDEAEYYCALWYSNLWVFGGGTKLTVL
   > CH1-CH2-CH3 (SEQ ID NO: 26) 
SSASTKGPSVFPLAPSSKSTSGGTAALGCLVEDYFPEPVTVSWNSGALTSGVHTFPAVLQ
SSGLYSLSSVVTVPSSSLGTQTYICNVNHKPSNTKVDDRVEPKSCDKTHTCPPCPAPPAA
GPSVFLFPPKPKDTLMISRTPEVTCVVVDVSHEDPEVKFNWYVDGVEVHNAKTKPREE
QYNSTYRVVSVLTVLHQDWLNGKEYKCKVSNKALAAPIEKTISKAKGQPREPQVCTL
PPSREEMTKNQVSLSCAVKGFYPSDIAVEWESNGQPENNYKTTPPVLDSDGSFFLVSRL
TVDKSRWQQGNVFSCSVMHEALHNHYTQKSLSLSPGK
Light Chain 1: VL-CL
   > VL (SEQ ID NO: 3) 
DIQMTQSPSSLSASVGDRVTITCKASQNVATHVGWYQQKPGKAPKRLIYSASYRYSGV
PSRFSGSGSGTEFTLTISNLQPEDFATYYCQQYNRYPYTFGQGTKLEIK
   > CL (SEQ ID NO: 24)
Light Chain 2: VH-CL
   > VH (SEQ ID NO: 4) 
EVQLVESGGGLVQPGGSLRLSCAASGFTFNTYAMNWVRQAPGKGLEWVARIRSKYNN
YATYYAASVKGRFTISRDDSKNSLYLQMNSLKTEDTAVYYCARHGNFGNSYVSWFAY
WGQGTLVTVSS
   > CL (SEQ ID NO: 27)

GQPKAAPSVTLFPPSSEELQANKATLVCLISDFYPGAVTVAWKADSSPVKAGVETTTPS
KQSNNKYAASSYLSLTPEQWKSHRSYSCQVTHEGSTVEKTVAPTECS

For the above four polypeptide chains, primers were designed. The corresponding coding genes were synthesized and connected into eukaryotic expression plasmids. The four recombinant expression plasmids obtained were co-transfected into HEK293F cells. After 5-7 days of cell culture, the supernatant with secreted antibodies was collected. The bispecific antibodies were purified from the supernatant using MabSelectSure affinity chromatography and SP cation exchange chromatography.

The obtained bispecific antibody was named as "GPRC5D×CD3 JNJ".

### Example 4: Binding between hybridoma cell culture supernatant and human GPRC5D detected via FACS

293T cells expressing human GPRC5D as described above was prepared into a suspension at concentration of 1 × 10⁵ cells/ml in PBS containing 2% FBS.

50 µl of cell suspension was added into each FACS tube (sample tube), then 50 µl of the supernatants of the hybridoma cell culture to be tested and the anti-hGPRC5D×CD3 bispecific antibody, GPRC5D×CD3 JNJ, as a positive control antibody were added followed by incubation at 4°C for 60 mins. 1 ml of FACS buffer was added to each FACS tube, centrifuged at 1200 rpm for 5 mins. The supernatant was discarded followed by washing three times. At the same time, control tube 1 (no supernatant of the culture and the secondary antibody mentioned below, adding only cell suspension) and control tube 2 (no supernatant of the culture, adding only cell suspension and the secondary antibody mentioned below) were included.

100 µl of FACS buffer was then added to each FACS tube for resuspension. 5 µl of PE-labeled, anti-murine Fc tagged secondary antibody (Biolegend, catalog number 409304) was added according to instructions, and incubated at 4°C in the dark for 30 mins. Then 1 ml of FACS buffer was added, and centrifuged at 1200 rpm at room temperature for 5 mins. The supernatant was discarded followed by washing three times.

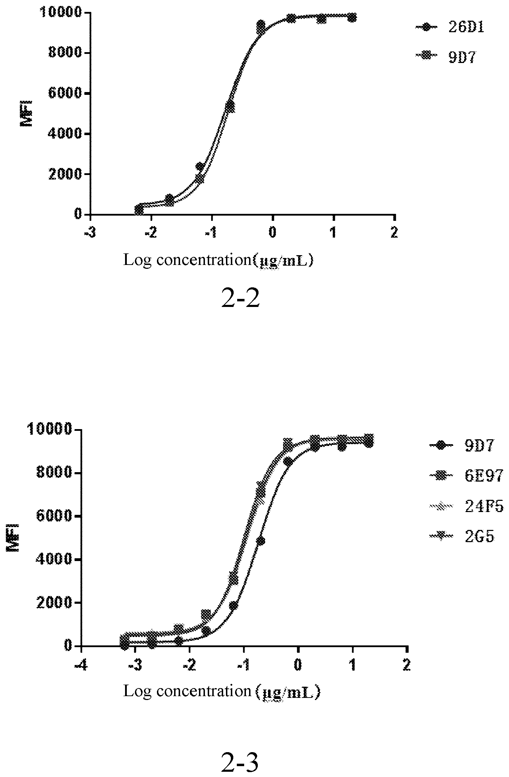

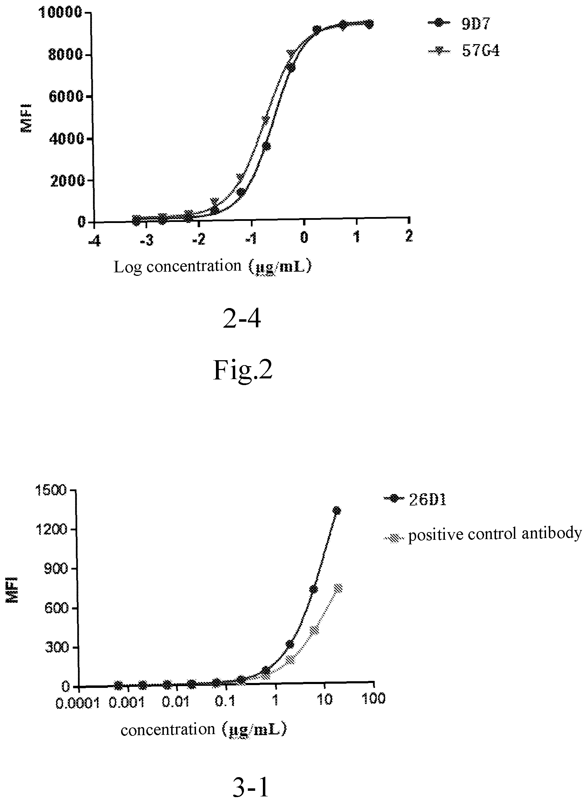

Again, 250 µl of FACS buffer was added to each FACS tube, resuspended and mixed. Then tested was performed on the machine. The results are shown in Tables 2-1 to 2-4 and Figures 2-1 to 2-4.

**Table 2-1**

| Sample shown with hybridoma cell number | 26D1 | Positive control antibody |
|---|---|---|
| EC50 (µg/mL) | 0.7881 | 1.684 |
| MFI. Max | 11491 | 10980 |

**Table 2-2**

| Sample shown with hybridoma cell number | 26D1 | 9D7 |
|---|---|---|
| EC50 (µg/mL) | 0.1688 | 0.1853 |
| MFI.Max | 9755 | 9785 |

**Table 2-3**

| Sample shown with hybridoma cell number | 9D7 | 6E97 | 24F5 | 2G5 |
|---|---|---|---|---|
| EC50 (µg/mL) | 0.1830 | 0.1108 | 0.1170 | 0.09987 |
| MFI.Max | 9383 | 9610 | 9515 | 9496 |

**Table 2-4**

| Sample shown with hybridoma cell number | 9D7 | 57G4 |
|---|---|---|
| EC50 (µg/mL) | 0.2793 | 0.1918 |
| MFI.Max | 9255 | 9227 |

### Example 5: Binding between hybridoma cell supernatant and monkey GPRC5D detected via FACS

According to the method described in Example 4, the binding between the culture supernatant of the hybridoma cells and monkey GPRC5D was detected, excpet that the human GPRC5D-expressing cells were replaced with 293T cells expressing monkey GPRC5D (Kyinno, catalog number KC-1586).

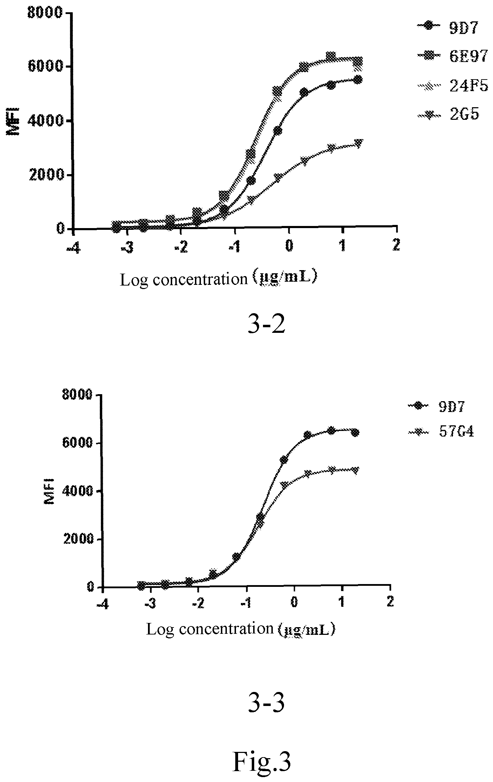

The results of FACS are shown in Tables 3-1 to 3-3 and Figures 3-1 to 3-3; the positive control antibody was GPRC5D×CD3 JNJ.

**Table 3-1**

| Sample shown with hybridoma cell number | 26D1 | Positive control antibody |
|---|---|---|
| EC50 (µg/mL) | 12.31 | 13.07 |
| MFI.Max | 1317 | 726 |

**Table 3-2**

| Sample shown with hybridoma cell number | 9D7 | 6E97 | 24F5 | 2G5 |
|---|---|---|---|---|
| EC5 (µg/mL) | 0.3761 | 0.2434 | 0.2683 | 0.4830 |
| MFI.Max | 5442 | 6294 | 6260 | 3050 |

**Table 3-3**

| Sample shown with hybridoma cell number | 9D7 | 57G4 |
|---|---|---|
| EC50 (µg/mL) | 0.2293 | 0.1820 |
| MFI.Max | 6447 | 4779 |

### Example 6: Binding between Hybridoma cell supernatant and different cells detected via FACS

According to the method described in Example 4, binding between the supernatant of the hybridoma cell culture and human GPRC5D was tested, except that the human GPRC5D-expressing cells were replaced with other cells as follows: NALM6 (Kyinno, catalog number KC-0626), NCI-H929 (Kyinno, catalog number KC-0629), MM1R (Kyinno, catalog number KC-0619), OPM2 (Kyinno, catalog number KC-0631) cells.

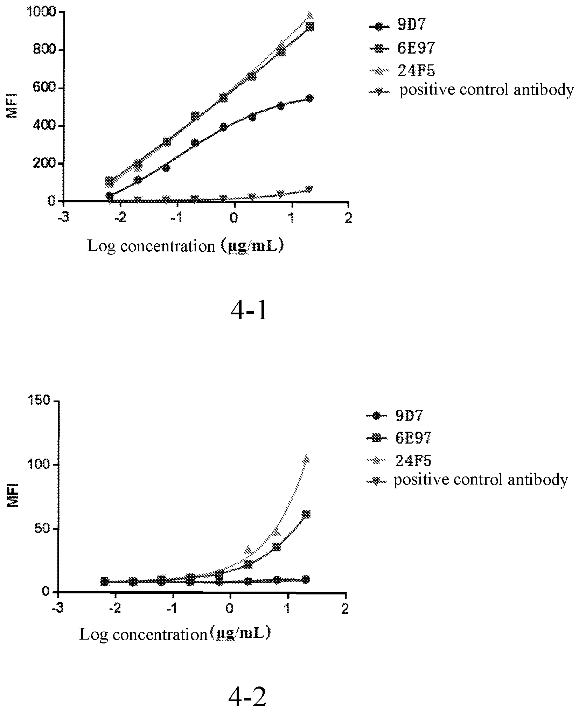

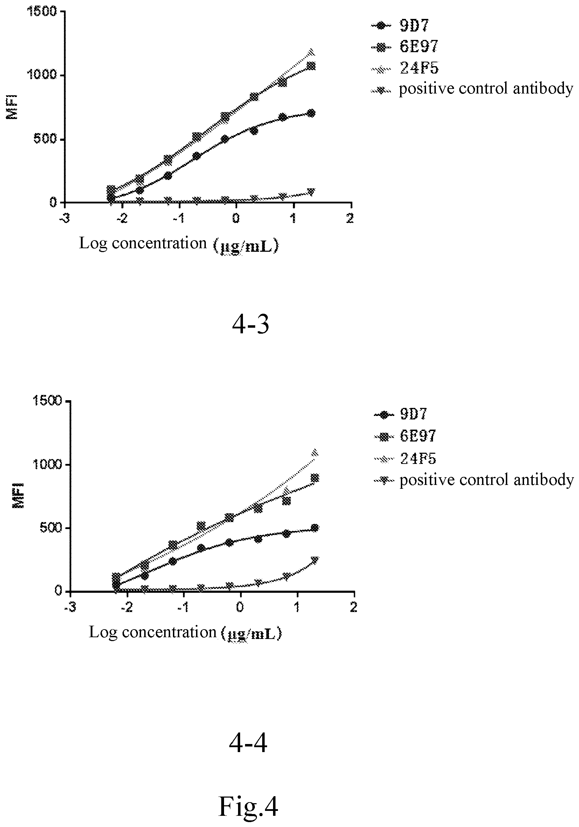

The results of FACS are shown in Table 4 and Figures 4-1 to 4-4; the positive control antibody was GPRC5D×CD3 JNJ.

**Table 4. FACS detection results of the binding between hybridoma cell supernatant and cells**

| MM1R cells | | | | |
|---|---|---|---|---|
| Sample shown with hybridoma cell number | 9D7 | 6E97 | 24F5 | Positive control antibody |
| EC50 (µg/mL) | 0.1038 | N/A | N/A | N/A |
| MFI.Max | 554 | 931 | 991 | 66.7 |

| Nalm6 cells | | | | |
|---|---|---|---|---|
| Sample shown with hybridoma cell number | 9D7 | 6E97 | 24F5 | Positive control antibody |
| EC50 (µg/mL) | N/A | N/A | N/A | N/A |
| MFI.Max | 10.8 | 62.4 | 106 | 10.6 |

| OPM2 cells | | | | |
|---|---|---|---|---|
| Sample shown with hybridoma cell number | 9D7 | 6E97 | 24F5 | Positive control antibody |
| EC50 (µg/mL) | 0.1766 | 0.2908 | 5.675 | N/A |
| MFI.Max | 705 | 1077 | 1192 | 80.6 |

| NCI-H929 cells | | | | |
|---|---|---|---|---|
| Sample shown with hybridoma cell number | 9D7 | 6E97 | 24F5 | Positive control antibody |
| EC50 (µg/mL) | 0.02096 | N/A | N/A | N/A |
| MFI.Max | 506 | 900 | 1104 | 243 |

### Example 7: Sequence identification of a murine antibody

The screened hybridoma cell line was selected. RNA was extracted from the monoclonal cells and reverse transcribed into cDNA. The cDNA was amplified, recovered from gel and connected into the sequencing vector for sequencing.

The sequences of the heavy and light chain variable regions of an exemplary murine antibody obtained from hybridoma cells are as follows (the CDRs defined by the KABAT method are indicated in bold-underlined font).
**A1:**
   >9D7-H (VH/HCDR-1/HCDR-2/HCDR-3: SEQ ID NO: 5/28/29/30) 
QVQLKESGPGLVKPSQSLSLTCTVTGYSITSDYAWNWIRQFPGNKLEWMGYISYSGSA
TYNPSLKSRISITRDTSKNQFFLQLNSMTTEDTATYYCARGGIAGRGRWGAMDYWG
QGTSVTVSS
   >9D7-L (VL/LCDR-1/LCDR-2/LCDR-3: SEQ ID NO: 6/31/32/33) 
DIVMTQAQKFMSTSVGDRVSVTCKASQNVGTNVAWYQNKPGQSPKALIYSASYRDS
GVPDRFTGRGSGTDFTLTISNVQSEDLAEYFCQQYKSYPLTFGAGTKLELK
**A2:**
   >26D1-H (VH/HCDR-1/HCDR-2/HCDR-3: SEQ ID NO: 7/35/36/37) 
QVQLKQSGAELAGPGASVKMSCKASGYTFTSYTIQWIKQRPGQGLEWIGYIIPSSGYT
NYNQKFKDKATLTADKSSNTAYMQLSSLTSEDSAVYYCANNYGNWGFTYWGQGTLV
TVSA
   >26D1-L (VL/LCDR-1/LCDR-2/LCDR-3: SEQ ID NO: 8/38/39/40) 
DILMTQSQKFMSTSVRDRVSISCKASQNVGSAVTWYQQKPGQSPRLLIYSASNRYTGV
PDRFTGSGSGTDFTLTISNVQSEDLADYFCQQYSNYPLTFGGGTKLEIK
**A3**
   >24F5-H (VH/HCDR-1/HCDR-2/HCDR-3: SEQ ID NO: 9/41/42/43 ) 
QVQLKESGPELVKPGASVKISCKASGYTFPYYVMHWVKQKPGQGLEWIGYINPYND
GTKYNEKFKGKAALTSDKSSSTAYMDLSSLTSEDSAVYYCARGGVRRYFDYWGQGT
TLTVSS
   >24F5-L <VL/LCDR-1/LCDR-2/LCDR-3: SEQ ID NO: 10/44/45/46) 
DIQMTQTPSSLSASLGERVSLTCRASQDIGSNLNWLQQEPDGTIKRLIYATSSLDSGVP
KRFSGSRSGSDYSLTISSLESEDFVDYYCLQYATFPYTFGGGTKLEIK
**A4**
   >6E97-H (VH/HCDR-1/HCDR-2/HCDR-3: SEQ ID NO: 11/47/42/43/) 
QVQLKESGPELVKPGASVKISCKASGYTFPYYVIHWVKQKPGQGLEWIGYINPYNDG
TKYNEKFKGKAALTSDKSSSTAYMELSSLTSEDSAVYYCARGGVRRYFDYWGQGTTL
TVSS
   >6E97-L (VL/LCDR-1/LCDR-2/LCDR-3: SEQ ID NO: 12/44/45/46)

QIVLTQSPSSLSASLGERVSLTCRASQDIGSNLNWLQQEPDGTIKRLISATSSLDSGVPK
RFSGSRSGSDYSLTISSLESEDFVDYFCLQYATFPYTFGGGTKLEIK

### Example 8: Preparation of a murine antibody-based chimeric antibody and humanized antibody

For the A1 antibody (murine anti-9D7), the following human germline sequences were selected as templates for the heavy chain and light chain: IGHV4-39 and IGKV1-39. Homology modeling was performed for the A1 antibody, and the structure of the Fab region was simulated. After homology modeling calculations, the predicted Fab structure of the A1 antibody was finally obtained.

By comparing and analyzing the predicted Fab structure and heavy chain with the IGHV4-39 sequence, all the murine amino acids in VH were replaced with the corresponding human amino acids in the IGHV4-39 template, except for the CDR regions and 2V, 49M, 68I, and 72R in VH which are the original murine amino acids.

By comparing and analyzing the predicted Fab structure and light chain with the IGKV1-39 sequence, all the murine amino acids of VL were replaced with the corresponding human amino acids in the IGKV1-39 template, except for the CDR regions and 42Q, 43S, 46A, and 60D of VL, which are the original murine amino acids. The humanized sequences obtained are as follows (the heavy chain and light chain CDRs defined by the KABAT definition method are shown in bold-underlined font):
The sequences of the humanized antibody are as follows:
>9D7-H humanized sequence (VH/HCDR-1/HCDR-2/HCDR-3: SEQ ID NO: 13/28/34/30) 
QVQLQESGPGLVKPSETLSLTCTVSGYSITSDYAWNWIRQPPGKGLEWMGYISYSGSA
TYSPSLKSRITISRDTSKNQFSLKLSSVTAADTAVYYCARGGIAGRGRWGAMDYWG
QGTTVTVSS
>9D7-L humanized sequence ( VL/LCDR-1/LCDR-2/LCDR-3: SEQ ID NO: 14/31/32/33)

DIQMTQSPSSLSASVGDRVTITCKASQNVGTNVAWYQQKPGQSPKALIYSASYRDSG
VPDRFSGSGSGTDFTLTISSLQPEDFATYYCQQYKSYPLTFGQGTRLEIK

For the A2 antibody (murine anti-26D1), the following human germline sequences were selected as templates for the heavy chain and light chain: IGHV1-46 and IGKV1-5. Homology modeling was performed for the A2 antibody, and the structure of the Fab region was simulated. After homology modeling calculations, the predicted Fab structure of the A2 antibody was finally obtained.

By comparing and analyzing the predicted Fab structure and heavy chain with the IGHV1-46 sequence, all the murine amino acids in VH were replaced with the corresponding human amino acids in the IGHV1-46 template, except for the CDR regions and 37I, 38K, 48I, 67R, 68A, 70L, 72A, 74K, 76S, 77N, 79A, and 98N in VH, which are the original murine amino acids.

By comparing and analyzing the predicted Fab structure and heavy chain with the IGKV1-5 sequence, all the murine amino acids in VL were replaced with the corresponding human amino acids in the IGKV1-5 template, except for the CDR regions and 45R, 60D, and 87F in VL, which are the original murine amino acids. The humanized sequences obtained are as follows (the heavy chain and light chain CDRs defined by the KABAT definition method are shown in bold-underlined font):
The sequences of the humanized antibody are as follows:
>26D1-H humanized sequence (VH/HCDR-1/HCDR-2/HCDR-3: SEQ ID NO: 15/35/36/37) 
QVQLVQSGAEVKKPGASVKVSCKASGYTFTSYTIQWIKQAPGQGLEWIGYIIPSSGYT
NYNQKFKDKATLTADKSSNTAYMELSSLTSEDTAVYYCANNYGNWGFTYWGQGTT
VTVSS
>26D1-L humanized sequence (VL/LCDR-1/LCDR-2/LCDR-3: SEQ ID NO: 16/38/39/40)

DIQMTQSPSTLSASVGDRVTITCKASQNVGSAVTWYQQKPGKAPRLLIYSASNRYTG
VPDRFSGSGSGTEFTLTISSLQPDDFATYFCQQYSNYPLTFGQGTRLEIK

For the A3 antibody (murine anti-24F5), the following human germline sequences were selected as templates for the heavy chain and light chain: IGHV1-46 and IGKV1-15. Homology modeling was performed for the A3 antibody, and the structure of the Fab region was simulated. After homology modeling calculations, the predicted Fab structure of the A3 antibody was finally obtained.

By comparing and analyzing the predicted Fab structure and heavy chain with the IGHV1-46 sequence, all the murine amino acids in VH were replaced with the corresponding human amino acids in the IGHV1-46 template, except for the CDR regions and 48I, 68A, 70L, 72S, 74K, and 79A in VH, which are the original murine amino acids.

By comparing and analyzing the predicted Fab structure and heavy chain with the IGKV1-15 sequence, all the murine amino acids in VL were replaced with the corresponding human amino acids in the IGKV1-15 template, except for the CDR regions and 36L, 42G, 43T, 44I, 46R, 66R, 69S, and 71Y in VL, which are the original murine amino acids. The humanized sequences obtained are as follows (the heavy chain and light chain CDRs defined by the KABAT method are shown in bold-underlined font ):
The sequences of the humanized antibody are as follows:
>24F5-H humanized sequence ( VH/HCDR-1/HCDR-2/HCDR-3: SEQ ID NO: 17/41/42/43 ) 
QVQLVQSGAEVKKPGASVKVSCKASGYTFPYYVMHWVRQAPGQGLEWIGYINPYN
DGTKYNEKFKGRATLTSDKSTSTAYMELSSLRSEDTAVYYCARGGVRRYFDYWGQ
GTTVTVSS
>24F5-L humanized sequence (VL/LCDR-1/LCDR-2/LCDR-3: SEQ ID NO: 18/44/45/46)

DIQMTQSPSTLSASVGDRVTITCRASQDIGSNLNWLQQKPGGTIKRLIYATSSLDSGVP
SRFSGSRSGSEYTLTISSLQPDDFATYYCLQYATFPYTFGQGTRLEIK

For the A4 antibody (murine anti-6E97), the following human germline sequences were selected as templates for the heavy chain and light chain: IGHV1-46 and IGKV1-15. Homology modeling was performed for the A4 antibody, and the structure of the Fab region was simulated. After homology modeling calculations, the predicted Fab structure of the A4 antibody was finally obtained.

By comparing and analyzing the predicted Fab structure and heavy chain with the IGHV1-46 sequence, all the murine amino acids in VH were replaced with the corresponding human amino acids in the IGHV1-46 template, except for the CDR regions and 48I, 68A, 70L, 72S, 74K, and 79A in VH, which are the original murine amino acids.

By comparing and analyzing the predicted Fab structure and heavy chain with the IGKV1-15 sequence, all the murine amino acids in VL were replaced with the corresponding human amino acids in the IGKV1-15 template, except for the CDR regions and 4L, 36L, 42G, 43T, 44I, 46R, 66R, 69S, and 71Y in VL, which are the original murine amino acids. The humanized sequences obtained are as follows (the heavy chain and light chain CDRs defined by the KABAT method are shown in bold-underlined font):
The sequences of the humanized antibody are as follows:
>6E97-H humanized sequence ( VH/HCDR-1/HCDR-2/HCDR-3: SEQ ID NO: 19/47/48/43) 
QVQLVQSGAEVKKPGASVKVSCKASGYTFPYYVIHWVRQAPGQGLEWIGYINPYNA
GTKYNEKFKGRATLTSDKSTSTAYMELSSLRSEDTAVYYCARGGVRRYFDYWGQG
TTVTVSS
>6E97-L humanized sequence <VL/LCDR-1/LCDR-2/LCDR-3: SEQ ID NO: 20/44/45/46)

DIQLTQSPSTLSASVGDRVTITCRASQDIGSNLNWLQQKPGGTIKRLISATSSLDSGVPS
RFSGSRSGSEYTLTISSLQPDDFATYYCLQYATFPYTFGQGTRLEIK

For the DNA sequences obtained via sequencing of the antibodies targeting GRRC5D with SEQ ID NO: 23 as the heavy chain constant region and SEQ ID NO: 24 as the light chain constant region, primers were redesigned to synthesize the corresponding genes of the chimeric antibody and humanized antibody. The synthesized sequences were connected into a eukaryotic expression vector, followed by transformed into DH5alpha competent cells, cultured in a constant temperature incubator at 37°C overnight. Monoclonal strains were selected for sequencing and identification. The strains with correct sequences were selected for culture. Plasmids were extracted and used to transfect mammalian 293F expression cells. The cells were placed in an incubator at 37°C, 5% CO2 and cultured for 7 days.

The supernatants were collected, centrifuged, filtered, and purified by protein G affinity chromatography. The purified antibodies were tested for purity by SDS-PAGE electrophoresis, and the concentration was tested by BCA protein detection kit. The antibodies were packaged and stored in refrigerator at -80°C for later use. The chimeric antibody was named as "abbreviation of mouse antibody (chi)" and the humanized antibody was named as " abbreviation of mouse antibody (hz)".
>CH1-CH3 heavy chain constant region (SEQ ID NO: 23)
>CL1 light chain constant region (SEQ ID NO: 24)

### Example 9: Binding between humanized antibody and human GPRC5D detected via FACS

The experimental process was as described in Example 4, wherein 293T cells expressing human GPRC5D (Kyinno, catalog number KC-1584) were used, and the antibodies to be tested, the positive control antibody GPRC5D×CD3 JNJ or the negative control antibody (hIgG1) were added at concentrations shown in Figure 4. Control tube 1 (no antibody and secondary antibody, adding only the cell suspension) and Control tube 2 (no antibody, adding only the cell suspension and the secondary antibody) were included.

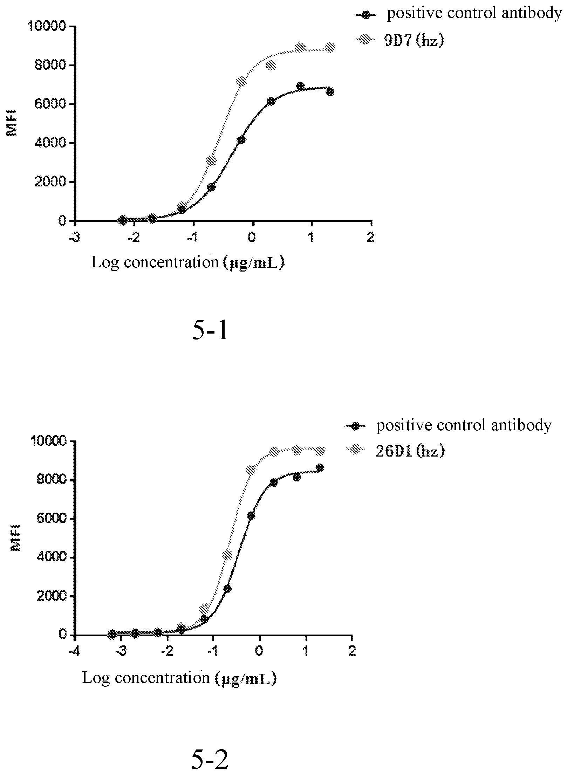

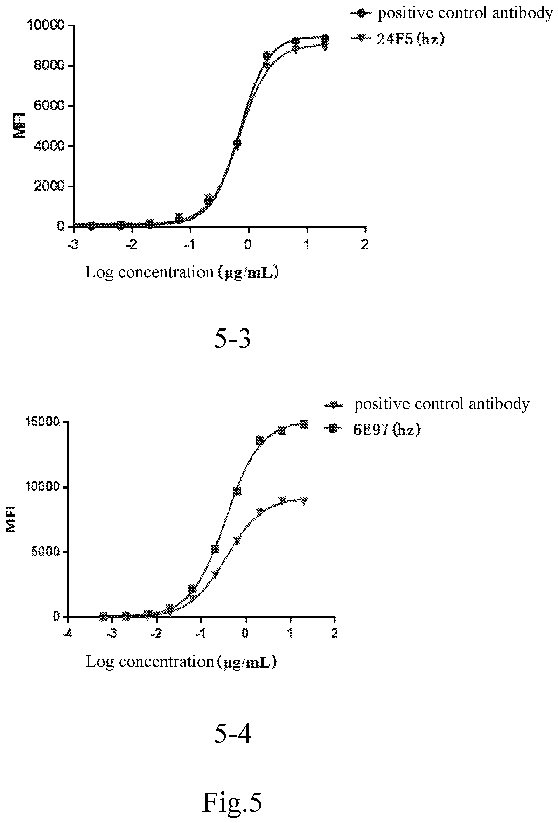

The results are shown in Table 5 and Figures 5-1 to 5-4.

**Table 5. FACS detection results of binding between humanized antibody and cells**

| Sample | Positive control antibody | 9D7(hz) |
|---|---|---|
| EC50 (µg/mL) | 0.4537 | 0.2830 |
| MFI.Max | 6590 | 8938 |
| | | |

| Sample | Positive control antibody | 26D 1 (hz) |
|---|---|---|
| EC50 (µg/mL) | 0.3552 | 0.228 |
| MFI.Max | 8650 | 9560 |
| | | |

| Sample | Positive control antibody | 24F5(hz) |
|---|---|---|
| EC50 (µg/mL) | 0.6986 | 0.6995 |
| MFI.Max | 9351 | 8908 |
| | | |

| Sample | Positive control antibody | 6E97(hz) |
|---|---|---|
| EC50 (µg/mL) | 0.3571 | 0.3508 |
| MFI.Max | 8941 | 14840 |

### Example 10: Binding between humanized antibody and monkey GPRC5D detected via FACS

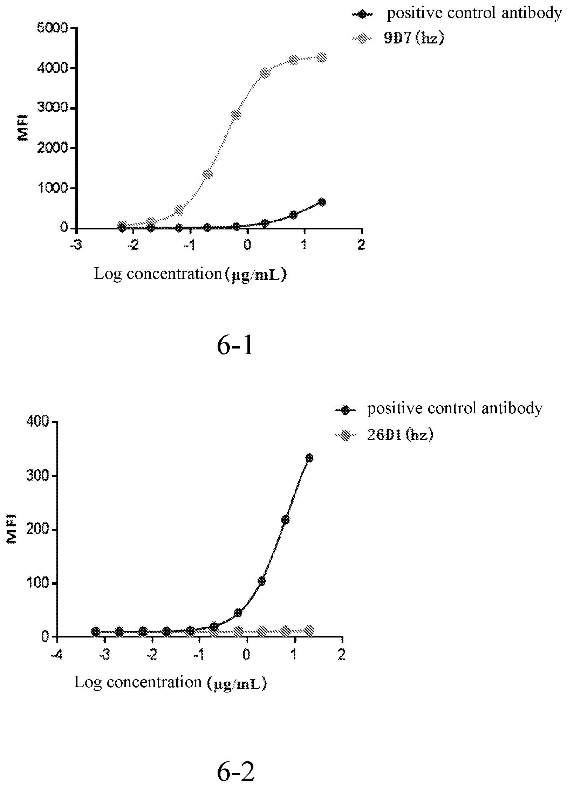

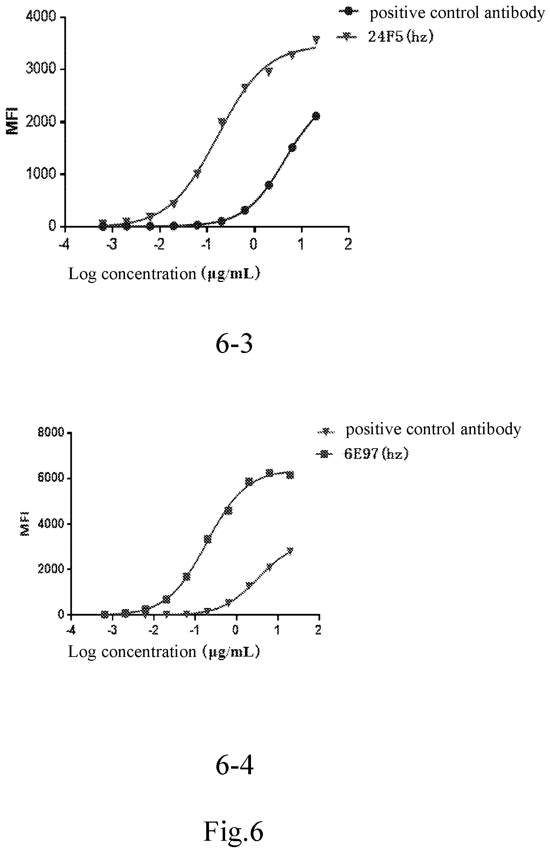

The experimental process was as described in Example 4. 293T cells expressing monkey (cyno) GPRC5D (Kyinno, catalog number KC-1586) were used, and the antibodies to be tested, the positive control antibody GPRC5D×CD3 JNJ or the negative control antibody (hIgG1) were added at concentrations shown in Figure 6. Control tube 1 (no antibody and secondary antibody, adding only cell suspension) and Control tube 2 (no antibody, adding only cell suspension and secondary antibody) were included.

The results are shown in Table 6 and Figures 6-1 to 6-4.

**Table 6. FACS detection results of binding between humanized antibodies and cells**

| Sample | Positive control antibody | 9D7(hz) |
|---|---|---|
| EC50 (µg/mL) | 13.96 | 0.3779 |
| MFI.Max | 656 | 4265 |
| | | |

| Sample | Positive control antibody | 26D 1 (hz) |
|---|---|---|
| EC50 (µg/mL) | N/A | N/A |
| MFI.Max | 334 | 12.3 |
| | | |

| Sample | Positive control antibody | 24F5(hz) |
|---|---|---|
| EC50 (µg/mL) | 4.389 | 0.1666 |
| MFI.Max | 2110 | 3558 |
| | | |

| Sample | Positive control antibody | 6E97(hz) |
|---|---|---|
| EC50 (µg/mL) | 3.265 | 0.1917 |
| MFI.Max | 2791 | 6246 |

### Example 11: Binding between humanized antibody and GPRC5A/B/C detected via FACS

The experimental process was as described in Example 4, cells expressing other members of G protein-coupled receptor class C group 5 as follows were used: 293 T cells expressing human GPRC5A, 293T-GPRC5A (Kyinno, catalog number KC-1888), 293T cells expressing human GPRC5B, 293T-GPRC5B (Kyinno, catalog number KC-1854), and 293T cells expressing human GPRC5C, 293T-GPRC5C (Kyinno, catalog number KC-1889). The antibodies to be tested, the positive control antibody GPRC5D×CD3 JNJ or the negative control antibody (hIgG1) were added at concentration of 2µg/mL. Control tube 1 (no antibody and secondary antibody, adding only cell suspension) and Control tube 2 (no antibody, adding only cell suspension and secondary antibody) were included.

The results are shown in Table 7.

**Table 7. FACS results of binding between humanized antibody and cells (µg/mL)**

| Cell | Positive control antibody | 9D7(hz) | 26D1(hz) | 24F5(hz) | 6E97(hz) | Control tube 1 | Control tube 2 |
|---|---|---|---|---|---|---|---|
| 293T-GPRC5A | 8.61 | 8.34 | 8.46 | 8.45 | 8.54 | 8.30 | 8.41 |
| 293T-GPRC5B | 8.44 | 8.54 | 8.59 | 8.58 | 8.62 | 8.55 | 8.26 |
| 293T-GPRC5C | 8.72 | 8.48 | 8.89 | 8.97 | 8.78 | 8.86 | 8.73 |

### Example 12: Binding between humanized antibody and tumor cell lines detected via FACS

The experimental process was as described in Example 4, and the cells used were replaced with multiple myeloma cells MM.1R (Kyinno, catalog number KC-0619) and MOLP8 (Kyinno, catalog number KC-0622). The antibodies to be tested, the positive control antibody GPRC5D×CD3 JNJ or the negative control antibody (hIgG1) were added. Control tube 1 (no antibody and secondary antibody, adding only cell suspension) and Control tube 2 (no antibody, adding only cell suspension and secondary antibody) were included.

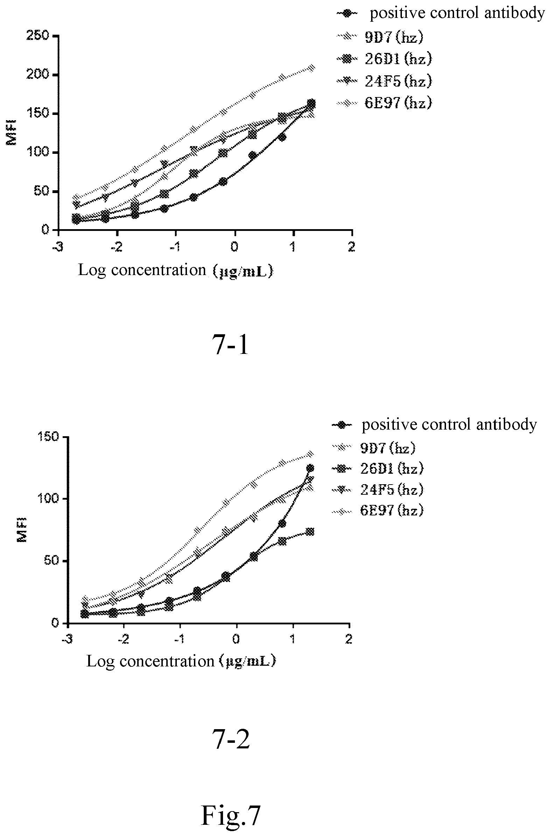

The results are shown in Table 8 and Figures 7-1 and 7-2.

**Table 8. FACS results of binding between humanized antibody and tumor cell lines**

| MM.1R cells | | | | | |
|---|---|---|---|---|---|
| Sample | Positive control antibody | 9D7(hz) | 26D1(hz) | 24F5(hz) | 6E97(hz) |
| EC50 (µg/mL) | 13.20 | 0.08998 | 0.5714 | 0.06819 | 0.1462 |
| MFI.Max | 164 | 150 | 185 | 177 | 246 |

| MOLP8 cells | | | | | |
|---|---|---|---|---|---|
| Sample | Positive control antibody | 9D7(hz) | 26D1(hz) | 24F5(hz) | 6E97(hz) |
| EC50 (µg/mL) | N/A | 0.3134 | 0.9644 | 0.5806 | 0.2563 |
| MFI.Max | 125 | 128 | 78 | 133 | 144 |

### Example 13: Affinity detection of humanized antibodies to GPRC5D protein

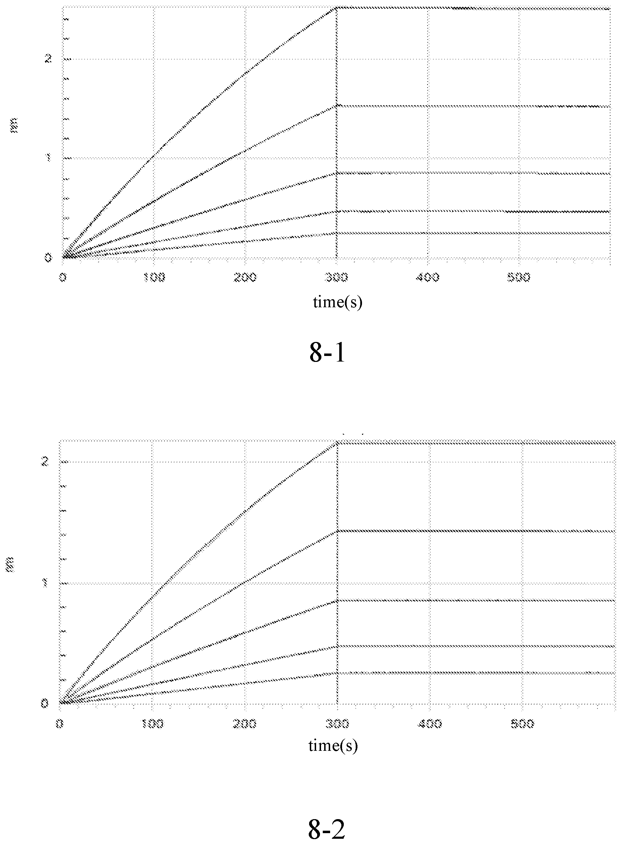

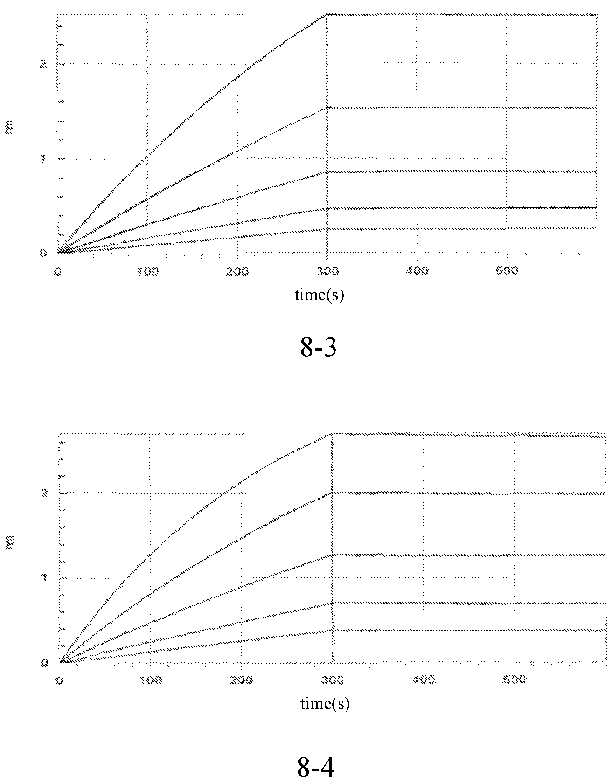

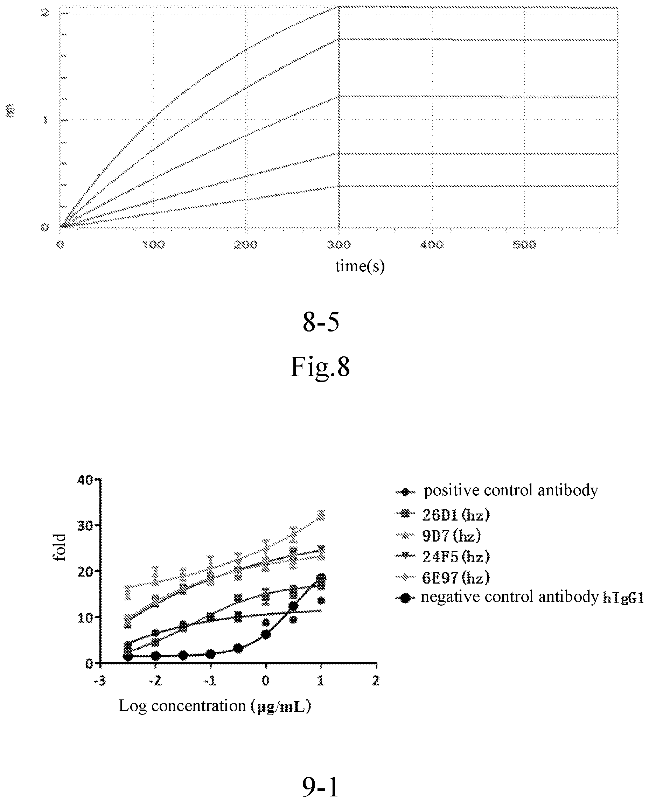

Antibodies to be tested were prepared into solutions at concentration of 10µg/mL using PBS buffer, and the antibodies were captured using the ARC chip according to Fortebio's instructions. The pre-prepared human GPRC5D proteins (KACTUS, catalog number GPR-HM05P) (protein starting at a highest concentration of 200nM, 2-fold diluted, 6 gradients) were flowed through the chip, with the following specific conditions: flow rate 30µl/min; antigen-antibody binding time 200 seconds, dissociation time 500 seconds. The measured results were fitted using the supporting software designed for the device to analyze the affinity between an antibody and an antigen. The results are shown in Table 9 and Figures 8-1 to 8-5.

**Table 9. Affinity test results of humanized antibody to GPRC5D protein**

| Antibody | KD(M) | kon(1/Ms) | kdis(1/s) |
|---|---|---|---|
| Positive control antibody | 9.47E-10 | 2.18E+04 | 2.06E-05 |
| 9D7(hz) | <1.0E-12 | 2.26E+04 | <1.0E-07 |
| 26D1(hz) | 1.70E-09 | 1.85E+04 | 3.15E-05 |
| 24F5(hz) | 1.36E-09 | 3.99E+04 | 5.42E-05 |
| 6E97(hz) | 4.58E-10 | 4.55E+04 | 2.08E-05 |

### Example 14: Detection of humanized antibody-mediated ADCC

20,000 reporter cells Jurkat-NFAT-luc-CD16-V158 (Kyinno, catalog number KC-1507), 20,000 wild-type NCI-H929 cells (CFSE-H929, Kyinno, catalog number KC-0629) or GPRC5D-knockout NCI-H929 cells (CFSE-H929GPRC5DKO, Kyinno, catalog number KC-2203), and the antibodies at different concentrations (10, 3.16, 1, 0.316, 0.1, 0.0316, 0.01, 0.00316 µg/mL) were added to each well of a U-bottom 96-well plate. The cells were cultured for 6 hrs, and the luciferase signals were detected.

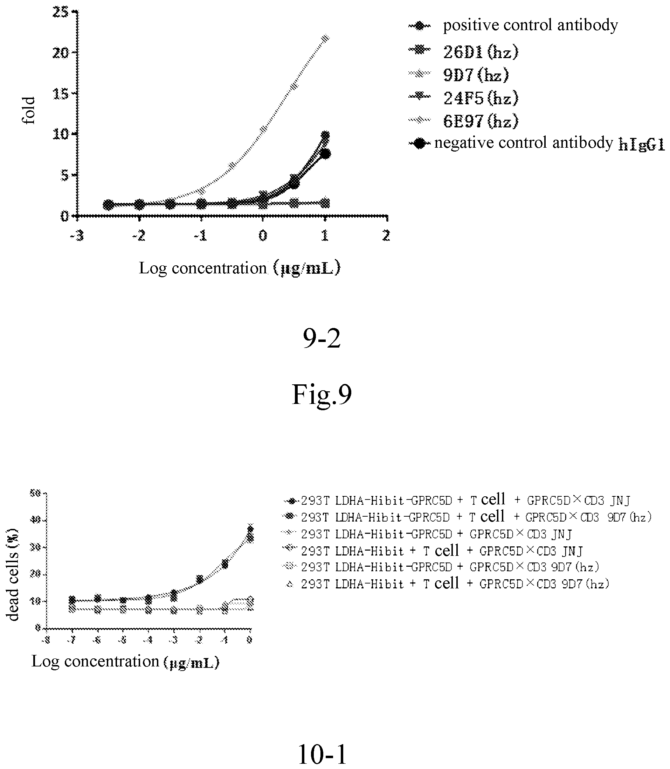

The results are shown in Table 10 and Figures 9-1 to 9-2; the positive control antibody is GPRC5D×CD3 JNJ.

**Table 10. The detection results of humanized antibody-mediated ADCC**

| Wild-type NCI-H929 | | | | | | |
|---|---|---|---|---|---|---|
| Sample | Positive control antibody | 26D 1 (hz) | 9D7(hz) | 24F5(hz) | 6E97(hz) | hIgG 1 |
| EC50 (µg/mL) | ∼ 2.130e-012 | 0.04520 | ∼ 6.750e-014 | ∼ 0.0 | ∼ 2.803e+014 | 3.198 |
| Top | 13.66664035 | 18.37682009 | 24.50338924 | 25.35668768 | 32.82225957 | 18.585700 |

| GPRC5D-knockout NCI-H929 | | | | | | |
|---|---|---|---|---|---|---|
| Sample | Positive control antibody | 26D 1 (hz) | 9D7(hz) | 24F5(hz) | 6E97(hz) | hIgG 1 |
| EC50 (µg/mL) | 9.705 | N/A | N/A | 14.70 | 2.600 | 5.173 |
| Top | 9.879115 | 1.555734 | 1.900409 | 8.872427 | 21.727390 | 7.622390 |

### Example 15: Acquisition of anti-CD3 antibodies and detection of target affinity

The heavy chain variable region sequence SEQ ID NO: 10 and the light chain variable region sequence SEQ ID NO: 5 of the murine antibody were obtained from patent US 10066015B2. The antibody sequences were humanized, and the humanized sequences obtained are as follows (the heavy chain and light chain CDRs defined by the KABAT method are shown in bold-underlined font):
>VH humanized sequence < VH/HCDR-1/HCDR-2/HCDR-3: SEQ ID NO: 21/49/50/51 ) 
QVQLVESGGGLVKPGGSLRLSCAASGFTFSTYAMNWVRQAPGKGLEWVGRIRSKYN
NYATYYADSVKDRFTISRDDAKNSLYLQMNSLRAEDTAVYYCARHGNFGNSYVSYF
AYWGQGTTVTVSS
>VL humanized sequence <VL/LCDR-1/LCDR-2/LCDR-3: SEQ ID NO: 22/52/53/54)

DIQMTQSPSSLSASVGDRVTITCRSSTGAVTTSNYANWVQQKPGKAPKALIGGTNKRA
PGVPSRFSGSLSGDDATLTISSLQPEDFATYYCALWYSNLWVFGQGTRLEIK

According to Example 8, the antibody comprising the above humanized sequences was obtained and named as SP34V1(hz). Similarly, a chimeric antibody was obtained using the heavy and light chain variable region sequences of the murine antibody in patent US 1006601 5B2 and named as SP34(chi).

The humanized antibody and the chimeric antibody were prepared into solutions at concentration of 10µg/mL using PBS buffer, and the antibodies were captured using the ARC chip according to Fortebio's instructions. The pre-prepared human CD3 protein (KACTUS, catalog number GPR-HM05P) (protein starting at a highest concentration of 200nM, 2-fold diluted, 6 gradients) were flowed through the chip, with the following specific conditions: flow rate 30µl/min; antigen-antibody binding time 200 seconds, dissociation time 500 seconds. The measured results were fitted using the supporting software designed for the device to analyze the affinity between an antibody and an antigen. The results are shown in Table 11.

**Table 11. Affinity results of antibodies to CD3 protein**

| Antibody | KD(M) | kon(1/Ms) | kdis(1/s) |
|---|---|---|---|
| SP34V1(hz) | 1.80E-08 | 4.42E+05 | 7.95E-03 |
| SP34(chi) | 5.46E-09 | 5.01E+05 | 2.74E-03 |

### Example 16: Construction of bispecific antibody

Bispecific antibodies were constructed as described in Example 3, except that the VH in Heavy Chain 1 and the VL in Light Chain 1 were replaced with the VH and VL of the humanized antibodies 9D7(hz), 26D1(hz), 24F5(hz) and 6E97(hz), respectively, and the VL in Heavy Chain 2 and the VH in Light Chain 2 were replaced with the humanized sequences directed to CD3 obtained in Example 15, respectively.

The anti-hGPRC5D×CD3 bispecific antibodies comprising the VH and VL of the humanized antibodies 9D7(hz), 26D1(hz), 24F5(hz) and 6E97(hz) and the above sequences were named as "GPRC5D×CD3 9D7(hz)", "GPRC5D×CD3 26D1(hz)", "GPRC5D×CD3 24F5(hz)" and "GPRC5D×CD3 26D1(hz)", respectively.

### Example 17: GPRC5D and CD3 dual antibody-mediated T cell killing

PBMC and CD3/CD28 magnetic beads were mixed at 2:1 ratio in RPMI1640 containing 10 ng/ml IL-2 and 10% FBS, and cultured 7 days for expansion to activate T cells.

Co-culture Experiment 1: 293T LDHA-Hibit-GPRC5D cells (Kyinno, catalog number KC-2151) were plated one day before (96-well plate), and 10,000 T cells as described above were added to each well, as well as different concentrations of positive control antibody GPRC5D×CD3 JNJ and the bispecific antibodies (1, 0.1, 0.01, 0.001, 0.0001, 0.00001, 0.000001, 0.0000001 µg/mL), and co-cultured for 18 hrs in RPMI1640 containing 10ng/ml IL-2 and 10% FBS. Nano-Glo^{®} HiBiT Extracellular Reagent (Promega, catalog number N2420) was used to detect the LDHA-HIBIT released by dead cells in the supernatant, thereby detecting the proportion of killing cells. This experiment set up two control groups for either of the two antibodies: adding 293T LDHA-Hibit-GPRC5D cells and antibodies, but not T cells; adding 293T LDHA-Hibit cells (Kyinno), T cells and antibodies, to exclude non-antibody-mediated killing and antibody-mediated non-T cell killing. The results are shown in Figure 10-1.

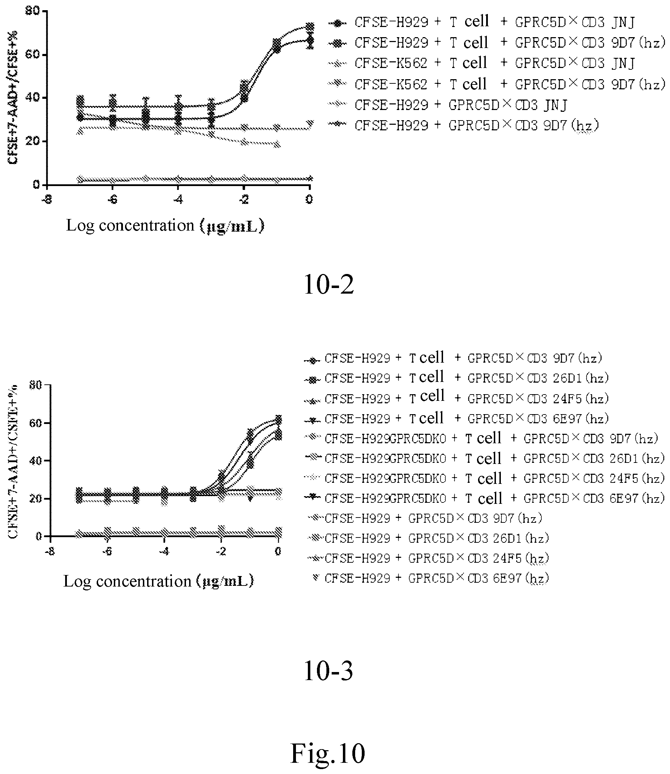

Co-culture Experiment 2: NCI-H929 (expressing GPRC5D) and K562 cells (not expressing GPRC5D) were stained with CFSE and named CFSE-H929 and CFSE-K562, respectively. 10,000 activated T cells, 3,000 CFSE-H929, and different concentrations of positive control antibody GPRC5D×CD3 JNJ and the bispecific antibodies (1, 0.1, 0.01, 0.001, 0.0001, 0.00001, 0.000001, 0.0000001 µg/mL) were added to each well of a U-bottom 96-well plate, and co-cultured for 18 hrs in RPMI1640 containing 10ng/ml IL-2 and 10% FBS. Then the cells were stained with 7-AAD and detected by flow cytometry. The ratio of CFSE+7-AAD+ cells in CSFE+ cells was determined as the cell killing ratio. This experiment set up two control groups for either of the two antibodies: adding CFSE-H929 cells and antibodies but not T cells; adding CFSE-K562, T cells and antibodies, to exclude non-antibody-mediated killing and antibody-mediated non-T cell killing. The results are shown in Figure 10-2.

Co-culture Experiment 3: Wild-type NCI-H929 (Kyinno, catalog number KC-0629) and GPRC5D-knockout NCI-H929 cells (Kyinno, catalog number KC-2203) were stained with CFSE and named CFSE-H929 and CFSE-H929GPRC5DKO, respectively. 10,000 activated T cells, 3,000 CFSE-H929, and different concentrations of positive control antibody GPRC5D×CD3 JNJ and the bispecific antibodies (1, 0.1, 0.01, 0.001, 0.0001, 0.00001, 0.000001, 0.0000001 µg/mL) were added to each well of a U-bottom 96-well plate and co-cultured in RPMI1640 containing 10ng/ml IL-2 and 10% FBS for 18 hrs. Then, the cells were stained with 7-AAD and detected by flow cytometry. The ratio of CFSE+7-AAD+ cells in CSFE+ cells was determined as the cell killing ratio. This experiment set up two control groups for each of the four antibodies: adding CFSE-H929 cells and antibodies, but not T cells; adding CFSE-H929GPRC5DKO, T cells and antibodies, to exclude non-antibody-mediated killing and antibody-mediated non-T cell killing. The results are shown in Figure 10-3.

The above description of the embodiments does not limit the invention. Those skilled in the art can make various changes or modifications based on the present disclosure, as long as they do not depart from the spirit of the present disclosure, and they should all fall within the scope of the claims of the present disclosure.

## Claims

1. An antibody or fragment thereof comprising a variable region of a heavy chain (VH) and a variable region of a light chain(VL), wherein the variable region of the heavy chain (VH) and the variable region of the light chain (VL) comprise a combination of H-CDR1, H-CDR2, H-CDR3 L-CDR1, L-CDR2 and L-CDR3 selected from:
(1) H-CDR1 comprising an amino acid sequence set forth in SEQ ID NO: 28, H-CDR2 comprising an amino acid sequence set forth in SEQ ID NO: 29, H-CDR3 comprising an amino acid sequence set forth in SEQ ID NO: 30, L-CDR1 comprising an amino acid sequence set forth in SEQ ID NO: 31, L-CDR2 comprising an amino acid sequence set forth in SEQ ID NO: 32, and L-CDR3 comprising an amino acid sequence set forth in SEQ ID NO: 33;
(2) H-CDR1 comprising an amino acid sequence set forth in SEQ ID NO: 28, H-CDR2 comprising an amino acid sequence set forth in SEQ ID NO: 34, H-CDR3 comprising an amino acid sequence set forth in SEQ ID NO: 30, L-CDR1 comprising an amino acid sequence set forth in SEQ ID NO: 31, L-CDR2 comprising an amino acid sequence set forth in SEQ ID NO: 32, and L-CDR3 comprising an amino acid sequence set forth in SEQ ID NO: 33;
(3) H-CDR1 comprising an amino acid sequence set forth in SEQ ID NO: 35, H-CDR2 comprising an amino acid sequence set forth in SEQ ID NO: 36, H-CDR3 comprising an amino acid sequence set forth in SEQ ID NO: 37, L-CDR1 comprising an amino acid sequence set forth in SEQ ID NO: 38, L-CDR2 comprising an amino acid sequence set forth in SEQ ID NO: 39, and L-CDR3 comprising an amino acid sequence set forth in SEQ ID NO: 40;
(4) H-CDR1 comprising an amino acid sequence set forth in SEQ ID NO: 41, H-CDR2 comprising an amino acid sequence set forth in SEQ ID NO: 42, H-CDR3 comprising an amino acid sequence set forth in SEQ ID NO: 43, L-CDR1 comprising an amino acid sequence set forth in SEQ ID NO: 44, L-CDR2 comprising an amino acid sequence set forth in SEQ ID NO: 45, and L-CDR3 comprising an amino acid sequence set forth in SEQ ID NO: 46;
(5) H-CDR1 comprising an amino acid sequence set forth in SEQ ID NO: 47, H-CDR2 comprising an amino acid sequence set forth in SEQ ID NO: 42, H-CDR3 comprising an amino acid sequence set forth in SEQ ID NO: 43, L-CDR1 comprising an amino acid sequence set forth in SEQ ID NO: 44, L-CDR2 comprising an amino acid sequence set forth in SEQ ID NO: 45, and L-CDR3 comprising an amino acid sequence set forth in SEQ ID NO: 46; or
(6) H-CDR1 comprising an amino acid sequence set forth in SEQ ID NO: 47, H-CDR2 comprising an amino acid sequence set forth in SEQ ID NO: 48, H-CDR3 comprising an amino acid sequence set forth in SEQ ID NO: 43, L-CDR1 comprising an amino acid sequence set forth in SEQ ID NO: 44, L-CDR2 comprising an amino acid sequence set forth in SEQ ID NO: 45, and L-CDR3 comprising an amino acid sequence set forth in SEQ ID NO: 46.

2. The antibody or fragment thereof according to claim 1, wherein the variable region of the heavy chain comprises a sequence selected from an amino acid sequence set forth in or an amino acid sequence having at least 75% identity with SEQ ID NO: 5, SEQ ID NO: 13, SEQ ID NO: 7, SEQ ID NO: 15, SEQ ID NO: 9, SEQ ID NO: 17, SEQ ID NO: 11 or SEQ ID NO: 19; and/or the variable region of the light chain comprises a sequence selected from an amino acid sequence set forth in or an amino acid sequence having at least 75% identity with SEQ ID NO: 6, SEQ ID NO: 14, SEQ ID NO: 8, SEQ ID NO: 16, SEQ ID NO: 10, SEQ ID NO: 18, SEQ ID NO: 12 or SEQ ID NO: 20.

3. The antibody or fragment thereof according to claim 1 or 2, wherein the variable region of the heavy chain and the variable region of the light chain of the antibody or fragment thereof comprise a sequence combination selected from the following:
(1) an amino acid sequence set forth in or an amino acid sequence having at least 75% identity with SEQ ID NO: 5; and an amino acid sequence set forth in or an amino acid sequence having at least 75% identity with SEQ ID NO: 6;
(2) an amino acid sequence set forth in or an amino acid sequence having at least 75% identity with SEQ ID NO: 13; and an amino acid sequence set forth in or an amino acid sequence having at least 75% identity with SEQ ID NO: 14;
(3) an amino acid sequence set forth in or an amino acid sequence having at least 75% identity with SEQ ID NO: 7; and an amino acid sequence set forth in or an amino acid sequence having at least 75% identity with SEQ ID NO: 8;
(4) an amino acid sequence set forth in or an amino acid sequence having at least 75% identity with SEQ ID NO: 15; and an amino acid sequence set forth in or an amino acid sequence having at least 75% identity with SEQ ID NO: 16;
(5) an amino acid sequence set forth in or an amino acid sequence having at least 75% identity with SEQ ID NO: 9; and an amino acid sequence set forth in or an amino acid sequence having at least 75% identity with SEQ ID NO: 10;
(6) an amino acid sequence set forth in or an amino acid sequence having at least 75% identity with SEQ ID NO: 17; and an amino acid sequence set forth in or an amino acid sequence having at least 75% identity with SEQ ID NO: 18;
(7) an amino acid sequence set forth in or an amino acid sequence having at least 75% identity with SEQ ID NO: 11; and an amino acid sequence set forth in or an amino acid sequence having at least 75% identity with SEQ ID NO: 12; and
(8) an amino acid sequence set forth in or an amino acid sequence having at least 75% identity with SEQ ID NO: 19; and an amino acid sequence set forth in or an amino acid sequence having at least 75% identity with SEQ ID NO: 20.

4. The antibody or fragment thereof according to any one of claims 1 to 3, wherein the antibody or fragment thereof is an anti-GPRC5D antibody or fragment thereof;
preferably, the antibody is in any form, for example, a murine antibody, a rabbit antibody, a monoclonal antibody, a chimeric antibody, a partially or fully humanized antibody; or, the fragment is a half antibody or an antigen-binding fragment of an antibody or a half antibody, for example, scFv, BsFv, dsFv, (dsFv)₂, Fab, Fab', F(ab')₂ or Fv;
more preferably, the antibody is IgG.

5. The antibody or fragment thereof according to any one of claims 1 to 4, wherein the antibody or fragment thereof further comprises a constant region;
preferably, the antibody or fragment thereof comprises a heavy chain and a light chain; more preferably, the antibody or fragment thereof comprises a constant region of a heavy chain of IgG, IgA, IgM, IgD or IgE and/or a constant region of a kappa or lambda light chain.

6. A bispecific antibody construct comprising a first binding domain that binds to G protein-coupled receptor class C group 5 member D (GPRC5D), and a second binding domain that binds to CD3 on the surface of a T cell, wherein:
the first binding domain comprises a variable region of a heavy chain (VH) and a variable region of a light chain (VL), wherein the variable region of the heavy chain (VH) and the variable region of the light chain (VL) comprise a CDR combination of H-CDR1, H-CDR2, H-CDR3, L-CDR1, L-CDR2 and L-CDR3 selected from the following:
(1) H-CDR1 comprising an amino acid sequence set forth in SEQ ID NO: 28, H-CDR2 comprising an amino acid sequence set forth in SEQ ID NO: 29, H-CDR3 comprising an amino acid sequence set forth in SEQ ID NO: 30, L-CDR1 comprising an amino acid sequence set forth in SEQ ID NO: 31, L-CDR2 comprising an amino acid sequence set forth in SEQ ID NO: 32, and L-CDR3 comprising an amino acid sequence set forth in SEQ ID NO: 33;
(2) H-CDR1 comprising an amino acid sequence set forth in SEQ ID NO: 28, H-CDR2 comprising an amino acid sequence set forth in SEQ ID NO: 34, H-CDR3 comprising an amino acid sequence set forth in SEQ ID NO: 30, L-CDR1 comprising an amino acid sequence set forth in SEQ ID NO: 31, L-CDR2 comprising an amino acid sequence set forth in SEQ ID NO: 32, and L-CDR3 comprising an amino acid sequence set forth in SEQ ID NO: 33;
(3) H-CDR1 comprising an amino acid sequence set forth in SEQ ID NO: 35, H-CDR2 comprising an amino acid sequence set forth in SEQ ID NO: 36, H-CDR3 comprising an amino acid sequence set forth in SEQ ID NO: 37, L-CDR1 comprising an amino acid sequence set forth in SEQ ID NO: 38, L-CDR2 comprising an amino acid sequence set forth in SEQ ID NO: 39, and L-CDR3 comprising an amino acid sequence set forth in SEQ ID NO: 40;
(4) H-CDR1 comprising an amino acid sequence set forth in SEQ ID NO: 41, H-CDR2 comprising an amino acid sequence set forth in SEQ ID NO: 42, H-CDR3 comprising an amino acid sequence set forth in SEQ ID NO: 43, L-CDR1 comprising an amino acid sequence set forth in SEQ ID NO: 44, L-CDR2 comprising an amino acid sequence set forth in SEQ ID NO: 45, and L-CDR3 comprising an amino acid sequence set forth in SEQ ID NO: 46;
(5) H-CDR1 comprising an amino acid sequence set forth in SEQ ID NO: 47, H-CDR2 comprising an amino acid sequence set forth in SEQ ID NO: 42, H-CDR3 comprising an amino acid sequence set forth in SEQ ID NO: 43, L-CDR1 comprising an amino acid sequence set forth in SEQ ID NO: 44, L-CDR2 comprising an amino acid sequence set forth in SEQ ID NO: 45, and L-CDR3 comprising an amino acid sequence set forth in SEQ ID NO: 46; and
(6) H-CDR1 comprising an amino acid sequence set forth in SEQ ID NO: 47, H-CDR2 comprising an amino acid sequence set forth in SEQ ID NO: 48, H-CDR3 comprising an amino acid sequence set forth in SEQ ID NO: 43, L-CDR1 comprising an amino acid sequence set forth in SEQ ID NO: 44, L-CDR2 comprising an amino acid sequence set forth in SEQ ID NO: 45, and L-CDR3 comprising an amino acid sequence set forth in SEQ ID NO: 46.

7. The bispecific antibody construct according to claim 6, wherein the variable region of the heavy chain in the first binding domain comprises a sequence selected from an amino acid sequence set forth in or an amino acid sequence having at least 75% identity with SEQ ID NO: 5, SEQ ID NO: 13, SEQ ID NO: 7, SEQ ID NO: 15, SEQ ID NO: 9, SEQ ID NO: 17, SEQ ID NO: 11 or SEQ ID NO: 19; and/or, the variable region of the light chain in the first binding domain comprises a sequence selected from an amino acid sequence set forth in or an amino acid sequence having at least 75% identity with SEQ ID NO: 6, SEQ ID NO: 14, SEQ ID NO: 8, SEQ ID NO: 16, SEQ ID NO: 10, SEQ ID NO: 18, SEQ ID NO: 12 or SEQ ID NO: 20.

8. The bispecific antibody construct according to claim 6 or 7, wherein the variable region of the heavy chain and the variable region of the light chain in the first binding domain comprise a sequence combination selected from the following:
(1) an amino acid sequence set forth in or an amino acid sequence having at least 75% identity with SEQ ID NO: 5; and an amino acid sequence set forth in or an amino acid sequence having at least 75% identity with SEQ ID NO: 6;
(2) an amino acid sequence set forth in or an amino acid sequence having at least 75% identity withSEQ ID NO: 13; and an amino acid sequence set forth in or an amino acid sequence having at least 75% identity with SEQ ID NO: 14;
(3) an amino acid sequence set forth in or an amino acid sequence having at least 75% identity with SEQ ID NO: 7; and an amino acid sequence set forth in or an amino acid sequence having at least 75% identity with SEQ ID NO: 8;
(4) an amino acid sequence set forth in or an amino acid sequence having at least 75% identity with SEQ ID NO: 15; and an amino acid sequence set forth in or an amino acid sequence having at least 75% identity with SEQ ID NO: 16;
(5) an amino acid sequence set forth in or an amino acid sequence having at least 75% identity with SEQ ID NO: 9; and an amino acid sequence set forth in or an amino acid sequence having at least 75% identity with SEQ ID NO: 10;
(6) an amino acid sequence set forth in or an amino acid sequence having at least 75% identity with SEQ ID NO: 17; and an amino acid sequence set forth in or an amino acid sequence having at least 75% identity with SEQ ID NO: 18;
(7) an amino acid sequence set forth in or an amino acid sequence having at least 75% identity with SEQ ID NO: 11; and an amino acid sequence set forth in or an amino acid sequence having at least 75% identity with SEQ ID NO: 12; or
(8) an amino acid sequence set forth in or an amino acid sequence having at least 75% identity with SEQ ID NO: 19; and an amino acid sequence set forth in or an amino acid sequence having at least 75% identity with SEQ ID NO: 20.

9. The bispecific antibody construct according to any one of claims 6 to 8, wherein the second binding domain comprises a variable region of a heavy chain (VH) and a variable region of a light chain (VL), wherein the variable region of the heavy chain (VH) and the variable region of the light chain (VL) comprise a CDR combination of H-CDR1, H-CDR2, H-CDR3; L-CDR1, L-CDR2, L-CDR3 selected from the following:
H-CDR1 comprising an amino acid sequence set forth in SEQ ID NO: 49, H-CDR2 comprising an amino acid sequence set forth in SEQ ID NO: 50, H-CDR3 comprising an amino acid sequence set forth in SEQ ID NO: 51, L-CDR1 comprising an amino acid sequence set forth in SEQ ID NO: 52, L-CDR2 comprising an amino acid sequence set forth in SEQ ID NO: 53, and L-CDR3 comprising an amino acid sequence set forth in SEQ ID NO: 54.

10. The bispecific antibody construct according to any one of claims 6 to 9, wherein the variable region of the heavy chain in the second binding domain comprises an amino acid sequence set forth in or an amino acid sequence having at least 75% identity with SEQ ID NO: 21; and the variable region of the light chain in the second binding domain comprises an amino acid sequence set forth in or an amino acid sequence having at least 75% identity with SEQ ID NO: 22.

11. The bispecific antibody construct according to any one of claims 6 to 10, wherein the bispecific antibody construct comprises four polypeptide chains:
1) Heavy Chain 1 comprising domains arranged as VH-CH1-CH2-CH3 from N-terminus to C-terminus;
2) Heavy Chain 2 comprising domains arranged as VL-CH1-CH2-CH3 from N-terminus to C-terminus;
3) Light Chain 1 comprising domains arranged as VL-CL from N-terminus to C-terminus;
4) Light Chain 2 comprising domains arranged as VH-CL from N-terminus to C-terminus;
wherein the VH and the VL in Heavy Chain 1 and Light Chain 1 are paired to form the first binding domain that binds to GPRC5D; the VL and the VH in Heavy Chain 2 and Light Chain 2 are paired to form the second binding domain that binds to CD3.

12. A nucleic acid molecule comprising a nucleotide sequence encoding a CDR of a heavy chain, a CDR of a light chain, a variable region of a light chain, a variable region of a heavy chain, a heavy chain or a light chain comprised in the antibody or fragment thereof according to any one of claims 1 to 5 or in the bispecific antibody construct according to any one of claims 6 to 11.

13. A vector comprising the nucleic acid molecule of claim 12.

14. A host cell comprising the nucleic acid molecule of claim 12 and/or the vector of claim 13.

15. A composition comprising the antibody or fragment thereof according to any one of claims 1 to 5, the bispecific antibody construct according to any one of claims 6 to 11, the nucleic acid molecule of claim 12, the vector of claim 13 and/or the host cell of claim 14;
preferably, the composition is a pharmaceutical composition, optionally further comprising a pharmaceutically acceptable excipient.

16. Use of the antibody or fragment thereof according to any one of claims 1 to 5, the bispecific antibody construct according to any one of claims 6 to 11, the nucleic acid molecule according to claim 12, the vector according to claim 13, the host cell according to claim 14 and/or the composition according to claim 15 in the preparation of a medicament for treating a disease, wherein the disease is tumor or cancer;
preferably, the disease is a tumor or cancer associated with GPRC5D expression (e.g., high expression), such as lymphoma or myeloma, particullarly multiple myeloma.

17. A method for treating a disease, comprising administering to a subject in need thereof the antibody or fragment thereof according to any one of claims 1 to 5, the bispecific antibody construct according to any one of claims 6 to 11, the nucleic acid molecule according to claim 12, the vector according to claim 13, the host cell according to claim 14 and/or the composition according to claim 15, wherein the disease is tumor or cancer;
preferably, the disease is a tumor or cancer associated with GPRC5D expression (e.g., high expression), such as lymphoma or myeloma, particullarly multiple myeloma;
preferably, the subject is a mammal; more preferably, the subject is human.

18. Use of the antibody or fragment thereof according to any one of claims 1 to 5, the bispecific antibody construct according to any one of claims 6 to 11, the nucleic acid molecule according to claim 12, the vector according to claim 13, the host cell according to claim 14 and/or the composition according to claim 15 in the preparation of an agent for diagnosing a disease or detecting the presence of GPRC5D antigen, wherein the disease is tumor or cancer;
preferably, the disease is a tumor or cancer associated with GPRC5D expression (e.g., high expression), such as lymphoma or myeloma, particullarly multiple myeloma.

19. A kit comprising the antibody or fragment thereof according to any one of claims 1 to 5, the bispecific antibody construct according to any one of claims 6 to 11, the nucleic acid molecule according to claim 12, the vector according to claim 13, the host cell according to claim 14 and/or the composition according to claim 15.
